# EUROPEAN PATENT APPLICATION

(11) **EP 1 557 474 A1**
(43) Date of publication of application: **27.07.2005**
(21) Application number: 04075185.1
(22) Date of filing: 21.01.2004
(51) Int. Cl.: C12Q 1/37, G01N 33/573, G01N 33/58, G01N 33/68

(54) **Method for determination of protein modifying or demodifying activity and suitable materials therefor**

(71) Applicant: Nederlandse Organisatie voor Toegepast-Natuurwetenschappelijk Onderzoek TNO, 2628 VK Delft (NL)
(72) Inventor: Verheijen, Johan Hendrikus, 2651 TX Berkel en Rodenrijs (NL); Hanemaaijer, Jan Roeland Occo, 2215 NP Voorhout (NL)
(74) Representative: Prins, Adrianus Willem, Mr. Ir.

(57) **Abstract**

Method for determining protein modifying activity or protein demodifying activity in a sample by providing a detection enzyme construct comprising at least one site of modification and at least one site of activation or inactivation, wherein activation or inactivation of the detection enzyme construct results from cleavage of the site of activation or inactivation by a protease, wherein the site of modification of the detection enzyme construct is capable of being modified by the protein modifying activity to be determined or demodified by the protein demodifying activity to be determined, and wherein modification or demodification of the site of modification affects cleavage of the site of activation or inactivation by the protease, contacting the sample with the detection enzyme construct under suitable conditions for the protein modifying or demodifying activity to be determined, contacting the detection enzyme construct with the protease under suitable conditions for activity of the protease, and determining the occurrence or level of detection enzyme activity. Active detection enzyme may be detectable by its conversion of a substrate of the detection enzyme to a reaction product. The detection enzyme construct may be derived from a protease (pro-)enzyme, like pro-urokinase or pro-caspase, and may comprise artificial modification site, activation cleavage site, and/or inactivation cleavage site.

SEQUENCE LISTING

<110>  Nederlandse Organisatie voor Toegepast Natuurwetenschappelijk Onderzoek
TNO

<120>  Method for determination of protein modifying or demodifying activity and
suitable materials therefor

<130>  P67104EP00

<140>  EP 04075185.1
<141>  2004-01-21

<160>  26

<170>  PatentIn version 3.1

<210>  1
<211>  21
<212>  DNA
<213>  Artificial Sequence

<220>
<223>  Primer

<400>  1
accatcgata accagccctg g                                                21


<210>  2
<211>  21
<212>  DNA
<213>  Artificial Sequence

<220>
<223>  Primer

<400>  2
ccgcctcgag gtcttttggc c                                                21


<210>  3
<211>  62
<212>  DNA
<213>  Artificial Sequence

<220>
<223>  Oligonucleotide

<400>  3
tcgaggcggc cgctcgggcg aagagtgtcc cttgccatta ttgggggaga attcaccacc      60

at                                                                     62


<210>  4
<211>  59
<212>  DNA
<213>  Artificial Sequence

<220>

<223>  Oligonucleotide

<400>  4
gatggtggtg aattctcccc caataatggc aagggacact cttcgcccga gcggccgcc      59


<210>  5
<211>  44
<212>  DNA
<213>  Artificial Sequence

<220>
<223>  Oligonucleotide

<400>  5
tcgaggcgcc ccctgggcat tattggcggg attaagggta ccat                      44


<210>  6
<211>  42
<212>  DNA
<213>  Artificial Sequence

<220>
<223>  Oligonucleotide

<400>  6
cgatggtacc cttaatcccg ccaataatgc ccagggggcg cc                        42


<210>  7
<211>  27
<212>  DNA
<213>  Artificial Sequence

<220>
<223>  Oligonucleotide

<400>  7
ggatcctgtc tcaatgccac agtccag                                         27


<210>  8
<211>  39
<212>  DNA
<213>  Artificial Sequence

<220>
<223>  Oligonucleotide

<400>  8
gatatcaagg aattcagtgg tgttgatgat gacatggcg                            39


<210>  9
<211>  27
<212>  DNA
<213>  Artificial Sequence

<220>
<223>  Oligonucleotide

<400> 9
gatcccttcg aagagcatcc cttggtc 27

<210> 10
<211> 27
<212> DNA
<213> Artificial Sequence

<220>
<223> Oligonucleotide

<400> 10
aattgaccaa gggatgctct tcgaagg 27

<210> 11
<211> 4
<212> PRT
<213> Artificial Sequence

<220>
<223> Peptide substrate

<220>
<221> MISC_FEATURE
<222> (4)..(4)
<223> "X" can be -pNA (para-nitro-anilide), -AFC (7-amino-4 trifluoro
methyl coumarine), -AMC (amino methyl coumarine), or any other
chromogenic or fluorogenic leaving group

<400> 11

Val Ala Asp Xaa
1

<210> 12
<211> 5
<212> PRT
<213> Artificial Sequence

<220>
<223> Peptide substrate

<220>
<221> MISC_FEATURE
<222> (5)..(5)
<223> "X" can be -pNA (para-nitro-anilide), -AFC (7-amino-4 trifluoro
methyl coumarine), -AMC (amino methyl coumarine), or any other
chromogenic or fluorogenic leaving group

<400> 12

Asp Glu Val Asp Xaa
1               5

<210>  13
<211>  5
<212>  PRT
<213>  Artificial Sequence

<220>
<223>  Peptide substrate

<220>
<221>  MISC_FEATURE
<222>  (5)..(5)
<223>  "X" can be -pNA (para-nitro-anilide), -AFC (7-amino-4 trifluoro
       methyl coumarine), -AMC (amino methyl coumarine), or any other
       chromogenic or fluorogenic leaving group

<400>  13

Val Glu Ile Asp Xaa
1               5

<210>  14
<211>  5
<212>  PRT
<213>  Artificial Sequence

<220>
<223>  Peptide substrate

<220>
<221>  MISC_FEATURE
<222>  (5)..(5)
<223>  "X" can be -pNA (para-nitro-anilide), -AFC (7-amino-4 trifluoro
       methyl coumarine), -AMC (amino methyl coumarine), or any other
       chromogenic or fluorogenic leaving group

<400>  14

Ile Glu Thr Asp Xaa
1               5

<210>  15
<211>  5
<212>  PRT
<213>  Artificial Sequence

<220>
<223>  Peptide substrate

<220>
<221>  MISC_FEATURE
<222>  (5)..(5)
<223>  "X" can be -pNA (para-nitro-anilide), -AFC (7-amino-4 trifluoro
       methyl coumarine), -AMC (amino methyl coumarine), or any other
       chromogenic or fluorogenic leaving group

<400>  15

Trp Glu His Asp Xaa
1               5


<210>  16
<211>  5
<212>  PRT
<213>  Artificial Sequence

<220>
<223>  Peptide substrate

<220>
<221>  MISC_FEATURE
<222>  (5)..(5)
<223>  "X" can be -pNA (para-nitro-anilide), -AFC (7-amino-4 trifluoro
       methyl coumarine), -AMC (amino methyl coumarine), or any other
       chromogenic or fluorogenic leaving group


<400>  16

Tyr Val Ala Asp Xaa
1               5


<210>  17
<211>  6
<212>  PRT
<213>  Artificial Sequence

<220>
<223>  Peptide substrate

<220>
<221>  MISC_FEATURE
<222>  (6)..(6)
<223>  "X" can be -pNA (para-nitro-anilide), -AFC (7-amino-4 trifluoro
       methyl coumarine), -AMC (amino methyl coumarine), or any other
       chromogenic or fluorogenic leaving group


<400>  17

Val Asp Val Ala Asp Xaa
1               5


<210>  18
<211>  14
<212>  PRT
<213>  Artificial Sequence

<220>
<223>  Part of modified PKA

<400>  18

Arg Pro Leu Gly Arg Arg Val Ser Leu Ala Ile Ile Gly Gly
1               5                   10

<210>  19
<211>  12
<212>  PRT
<213>  Artificial Sequence

<220>
<223>  Part of UK LH sequence

<400>  19

Arg Pro Leu Gly Ile Ile Gly Gly Ile Lys Gly Thr
1               5                   10


<210>  20
<211>  47
<212>  PRT
<213>  Artificial Sequence

<220>
<223>  Part of the pro-Caspase-3 sequence

<400>  20

Leu Thr Gly Lys Pro Lys Leu Phe Ile Ile Gln Ala Cys Arg Gly Thr
1               5                   10                  15


Glu Leu Asp Cys Gly Ile Glu Thr Asp Ser Gly Val Asp Asp Asp Met
            20                  25                  30


Ala Cys His Lys Ile Pro Val Asp Ala Asp Phe Leu Tyr Ala Tyr
        35                  40                  45


<210>  21
<211>  15
<212>  PRT
<213>  Artificial Sequence

<220>
<223>  Part of the mutated pro-Caspase-3 sequence

<400>  21

Gly Ile Glu Thr Gly Ser Asp Ile Lys Glu Phe Ser Gly Val Asp
1               5                   10                  15


<210>  22
<211>  45
<212>  DNA
<213>  Artificial Sequence

<220>
<223>  Part of the pro-Caspase-3 codingsequence

<400>  22

ggcattgaga caggatccga tatcaaggaa ttcagtggtg ttgat                    45

<210>  23
<211>  9
<212>  PRT
<213>  Artificial Sequence

<220>
<223>  Kinase recognition and protease activation site

<400>  23

Ser Leu Arg Arg Ala Ser Leu Gly Gln
1               5

<210>  24
<211>  27
<212>  DNA
<213>  Artificial Sequence

<220>
<223>  Oligonucleotide coding for kinase and activation site

<400>  24
gatcccttcg aagagcatcc cttggtc                                        27

<210>  25
<211>  27
<212>  DNA
<213>  Artificial Sequence

<220>
<223>  Reversed oligonucleotide coding for kinase and activation site

<400>  25
ggaagcttct cgtagggaac cagttaa                                        27

<210>  26
<211>  24
<212>  PRT
<213>  Artificial Sequence

<220>
<223>  Modified Caspase sequence with kinase recognition and protease
       activation site

<400>  26

Leu Asp Cys Gly Ile Glu Thr Gly Ser Leu Arg Arg Ala Ser Leu Gly
1               5                   10                  15

Gln Phe Ser Gly Val Asp Asp Asp
            20

## Description

### Field of the invention

This invention is in the field of determining, assaying or quantifying protein modifying activity or activity removing modifications from proteins (further referred to herein as protein demodification or demodifying activity).

More particularly, the invention relates to a method for determining presence or level of activity in a sample of an enzyme or enzymes catalyzing the modification of proteins or catalyzing the removal of modification of proteins.

The invention also concerns a detection enzyme construct that can be modified by the activity to be determined or from which a modification can be removed by the protein demodification activity to be determined and in unmodified form or modified form respectively in modified form or demodified form can be activated by limited proteolysis into a measurable active enzyme.

The invention also concerns an assay kit containing such a detection enzyme construct, as well as a cell expressing or containing the detection enzyme construct and an expression vector coding for the detection enzyme construct.

### Background of the invention

Applicant's European patent EP 0 691 409 B1 discloses a method for determining a protease, or its precursor after activation, in a sample, comprising incubating the sample with a substrate of said protease and determining proteolytic cleavage of said substrate, wherein said substrate is a modified proenzyme containing a recognition site which is cleavable by said protease and derived from a proenzyme by providing it with a recognition site which is cleavable by a protease different from the one which activates the unmodified proenzyme. The method allows to determine a specific proteolytic activity in a sample, for example the activity of an aspartic protease or a metalloprotease. The modified proenzyme may for example be derived from a serine protease, such as pro-urokinase. The modification of the proenzyme may consist of a replacement of the natural activation site of pro-urokinase (which is recognized by plasmin, the natural activator of pro-urokinase) by a new activation site specifically recognized by the protease to be determined.

Applicant's copending European patent application No. 03 075 319.8, filed January 31, 2003, discloses a similar method for determining a protease, wherein the modified proenzyme is specifically derived from a caspase.

Whereas the above mentioned documents are concerned with a method of determining a proteolytic activity, the present invention addresses the problem of determining a protein modifying or demodifying activity.

Many proteins have been found to contain modifications that result from physical, chemical or enzymatic processes acting on its aminoacid residues resulting in modified amino acid residues. Such modifications can be introduced during protein synthesis on the growing peptide chain or be introduced in the completed and properly folded protein after synthesis.

Examples of protein modifications and activities leading to modifications or removing modifications are summarized in Table I. They generally concern the introduction or removal of a chemical moiety, usually a small moiety like a methyl group, a phosphate group, a hydroxyl group, etc., but the chemical moiety may also be a large group, such as a carbohydrate side chain. The modification may comprise the formation of a complex with another protein (the same or different) or, more in general, with other (macro)molecules, including for example complex formation with nucleic acid or with cofactors of the protein in question. Therefore, the covalent attachment of a chemical moiety or molecule is not a prerequisite. Usually, modification or demodification is catalyzed by a specific enzyme that recognizes particular sites (sequences of aminoacid residues) and directs the modification or demodification to particular amino acid residues.

Recently is has become clear that modification of a protein can greatly influence its properties.

A particularly interesting modification is phosphorylation of a protein. The introduction of one or more phosphate groups into proteins can greatly influence their properties such as enzymatic activity, affinity for receptors, physical form, monomer, dimer, multimer. Introduction and removal of phosphate groups into proteins appears to be an important mechanism of signal transduction involved in critical processes. Introduction of phosphate groups into proteins is catalyzed by specific enzymes called protein kinases of which over 500 are known at present. Removal of phosphate groups from proteins is brought about by enzymes known as protein phosphatases of which over 100 different examples have been described. Both protein kinases and protein phosphatases are very interesting targets for development of drugs to diseases such as cancer, inflammatory diseases such as rheumatoid arthritis and diabetic complication.

Measurement of the presence or level of protein modifying or demodifying activities, particularly the activity of enzymes catalyzing protein modifying or demodifying reactions, is of considerable importance in basic academic research, drug discovery research, drug development and might also have applications in diagnostic or prognostic methods or as (surrogate) marker to follow or monitor (experimental) therapy.

Measurement and quantification of protein modifying or demodifying activity is in general not easy and requires often a variety of complicated, time-consuming and expensive techniques.

US 2002/0172991 A1 discloses a method for measuring protein kinase activity. The method uses the fact that the reaction catalyzed by the protein kinase uses ATP as a donor for the phosphate group. The method measures the change of ATP concentration using a bioluminescence reaction which involves luciferin and a luciferase. This method has the disadvantage that it is indirect, it does not measure the kinase activity itself, but the consumption of ATP. Further, the method is restricted to the measurement of ATP-dependent protein kinases and is not applicable to the measurement of other modifying or demodifying activities.

### Summary of the invention

This invention provides in a first aspect a method for determining a protein modifying activity or protein demodifying activity in a sample, the method comprising
providing a detection enzyme construct comprising at least one site of modification and at least one site of activation or inactivation, wherein activation or inactivation of the detection enzyme construct results from cleavage of said site of activation or inactivation by a protease, wherein said site of modification of the detection enzyme construct is capable of being modified by the protein modifying activity to be determined or demodified by the protein demodifying activity to be determined, and wherein modification or demodification of said site of modification affects cleavage of the said site of activation or inactivation by the said protease,
contacting the sample with the said detection enzyme construct under suitable conditions for the protein modifying or demodifying activity to be determined,
contacting the detection enzyme construct with the said protease under suitable conditions for activity of the said protease, and
determining the occurrence or level of detection enzyme activity.

In one group of preferred embodiments of the invention, said detection enzyme construct comprises a site of modification and a site of activation, wherein activation of the detection enzyme construct results from cleavage of said site of activation by an activation protease to form active detection enzyme, wherein said site of modification of the detection enzyme construct is capable of being modified by the protein modifying activity to be determined or demodified by the protein demodifying activity to be determined, and wherein modification or demodification of said site of modification determines whether said activation protease can cleave the said site of activation,
and the method comprises contacting the detection enzyme construct with the said activation protease under suitable conditions for activity of the said activation protease.

In another group of preferred embodiments of the invention, said detection enzyme construct comprises a site of modification, a site of activation and a site of inactivation, wherein activation of the detection enzyme construct results from cleavage of said site of activation by an activation protease to form active detection enzyme, and inactivation of the detection enzyme construct or detection enzyme results from cleavage of said site of inactivation by a deactivation protease, wherein said site of modification of the detection enzyme construct is capable of being modified by the protein modifying activity to be determined or demodified by the protein demodifying activity to be determined, and wherein modification or demodification of said site of modification determines whether said activation protease can cleave the said site of activation or said inactivation protease can cleave the said site of inactivation,
and the method comprises contacting the detection enzyme construct with the said activation protease under suitable conditions for activity of the said activation protease, and before, after, or simultaneously therewith, contacting the detection enzyme construct or detection enzyme with the said inactivation protease under suitable conditions for activity of the said inactivation protease.

In this group of embodiments of the invention, it is preferred that modification or demodification of said site of modification determines whether said inactivation protease can cleave the said site of inactivation.

In preferred embodiments of this invention, active detection enzyme is detectable by its conversion of a substrate of the detection enzyme to a reaction product and detection enzyme activity is determined by contacting the detection enzyme construct or detection enzyme with said substrate of the detection enzyme and determining the occurrence or level of conversion of said substrate.

Preferably, said detection enzyme construct is derived from a protease enzyme or protease pro-enzyme. More preferably, said detection enzyme construct is derived from a serine protease enzyme or serine protease pro-enzyme or from a cysteine protease enzyme or cysteine protease pro-enzyme. In some particular preferred embodiments, said detection enzyme construct is derived from a pro-enzyme selected from the group consisting of pro-urokinase, pro-granzyme, pro-caspase-3 and pro-caspase-7. In some most preferred embodiments, said detection enzyme construct is derived from pro-urokinase. In some other most preferred embodiments, said detection enzyme construct is derived from a pro-caspase, in particular pro-caspase-3.

Preferably, said detection enzyme construct contains an artificial modification site, or an artificial activation cleavage site, or an artificial inactivation cleavage site, or any combination thereof, and optionally further changes, introduced into a protease enzyme or protease pro-enzyme in such a way that modification or demodification of said modification site affects activatability of the detection enzyme construct by treatment with said activation protease or inactivatability of the detection enzyme construct or detection enzyme by treatment with said inactivation protease.

The activation protease may be selected from the group consisting of (a) the natural activation protease of said protease pro-enzyme, (b) a protease specifically cleaving an artificial activation cleavage site introduced into said protease pro-enzyme, (c) an aminopeptidase, and (d) a carboxypeptidase.

Similarly, the inactivation protease, which may be the same or different from the activation protease, may be selected from the group consisting of (a) a natural inactivation protease of said protease enzyme, (b) a protease specifically cleaving an artificial inactivation cleavage site introduced into said protease pro-enzyme, (c) an aminopeptidase, and (d) a carboxypeptidase.

Usually, said protein modifying activity or protein demodifying activity is an enzymatic activity. Preferably, said enzymatic activity is selected from the group consisting of of protein kinase activity, protein phosphatase activity, protein hydroxylase activity, protein glycosyltransferase activity or protein prenyltransferase activity.

In methods according to the invention for determining a protein demodifying activity, the sample would usually be contacted with pre-modified detection enzyme construct.

The sample may be contacted with a purified or partially purified detection enzyme construct, or with a cell expressing or containing the detection enzyme construct, or with the content of such a cell or of a specific compartment of such a cell, or with the culture medium of such a cell.

The sample may be contacted with a cell expressing or containing the detection enzyme construct and then either the cell as a whole, or the content of the cell or of a specific compartment of the cell, or the culture medium of the cell is contacted with the activation and/or inactivation protease and detection enzyme substrate.

In another aspect, the invention provides a detection enzyme construct useful for determining a protein modifying activity or protein demodifying activity in a sample, comprising at least one site of modification and at least one site of activation or inactivation, wherein activation or inactivation of the detection enzyme construct results from cleavage of said site of activation or inactivation by a protease, wherein said site of modification of the detection enzyme construct is capable of being modified by the protein modifying activity to be determined or demodified by the protein demodifying activity to be determined, and wherein modification or demodification of said site of modification affects cleavage of the said site of activation or inactivation by the said protease.

In a further aspect, the invention provides a cell expressing or containing a detection enzyme construct as defined herein.

In a further aspect, the invention provides a kit for determining a protein modifying activity or a protein demodifying activity in a sample, comprising a detection enzyme construct or a cell as defined herein, and further comprising at least one member of the group consisting of activation protease, inactivation protease, substrate of the detection enzyme, a standard composition, a buffer composition, an antibody composition, a stabilizing composition, an assay plate or container, and instructions for use.

The invention also provides an expression vector coding for a detection enzyme construct as defined herein.

### Brief description of the drawings

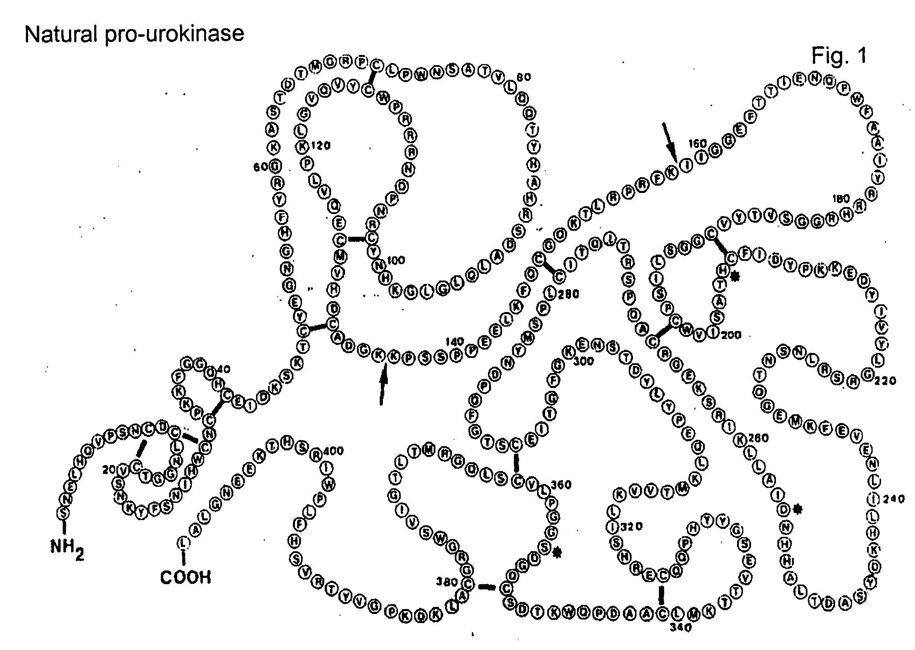

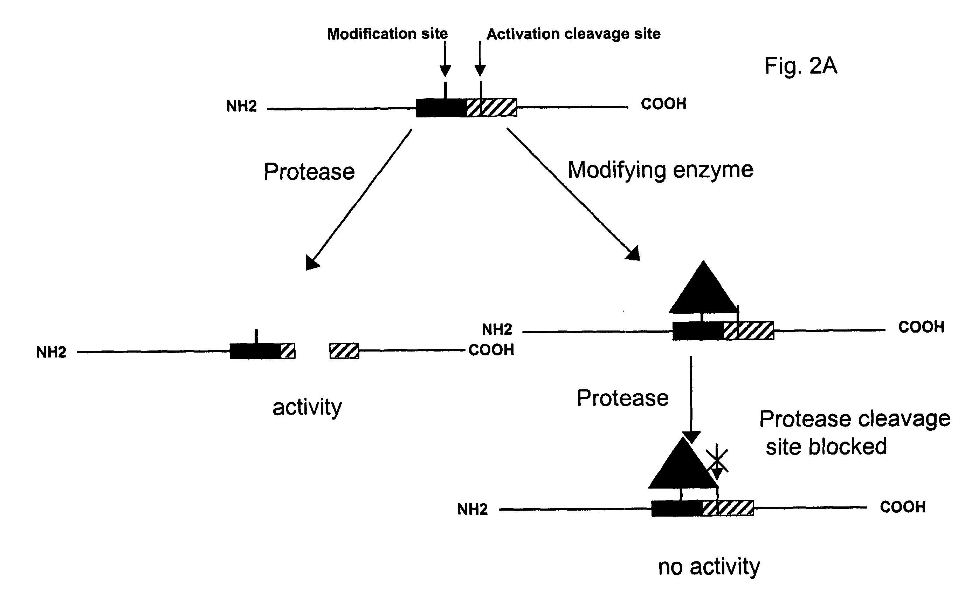

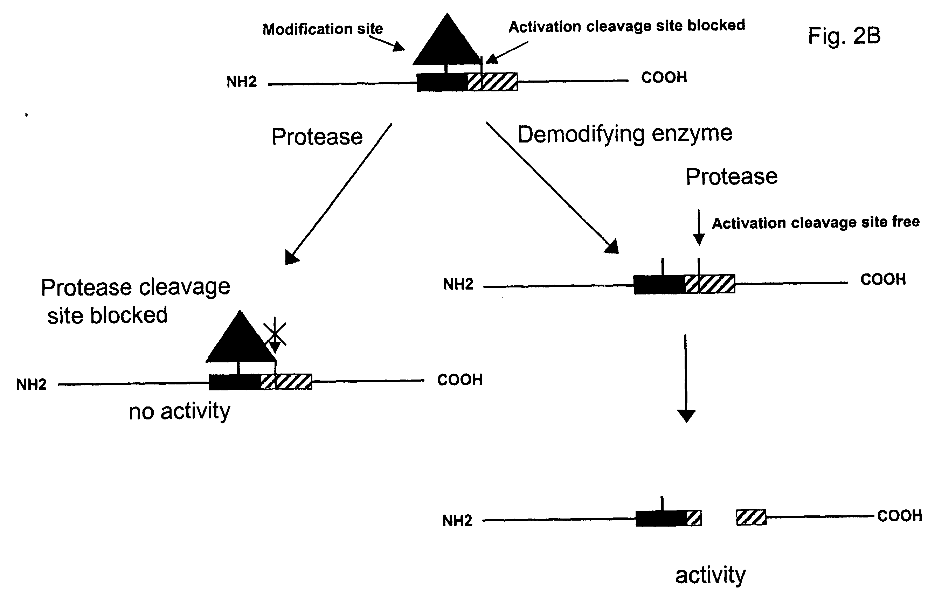

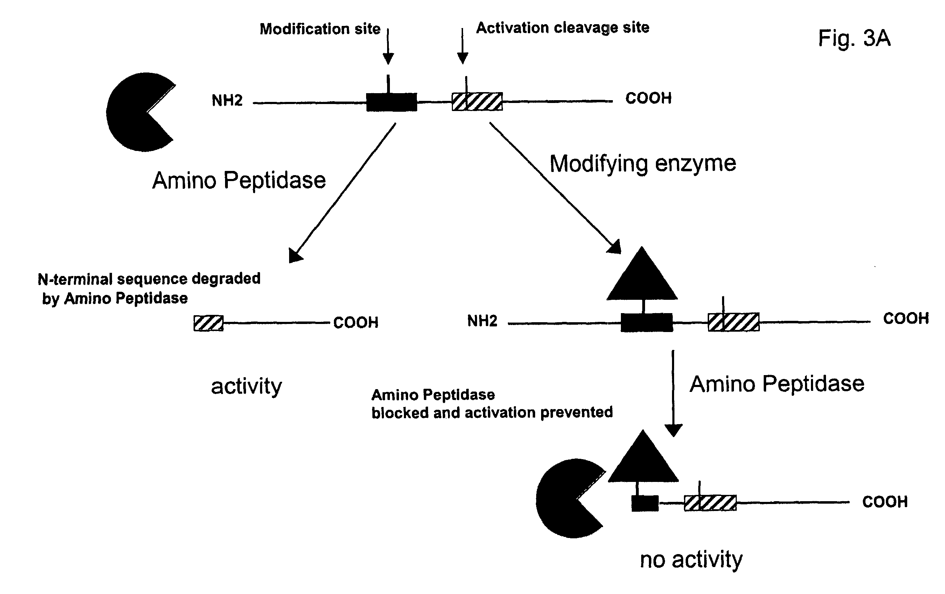

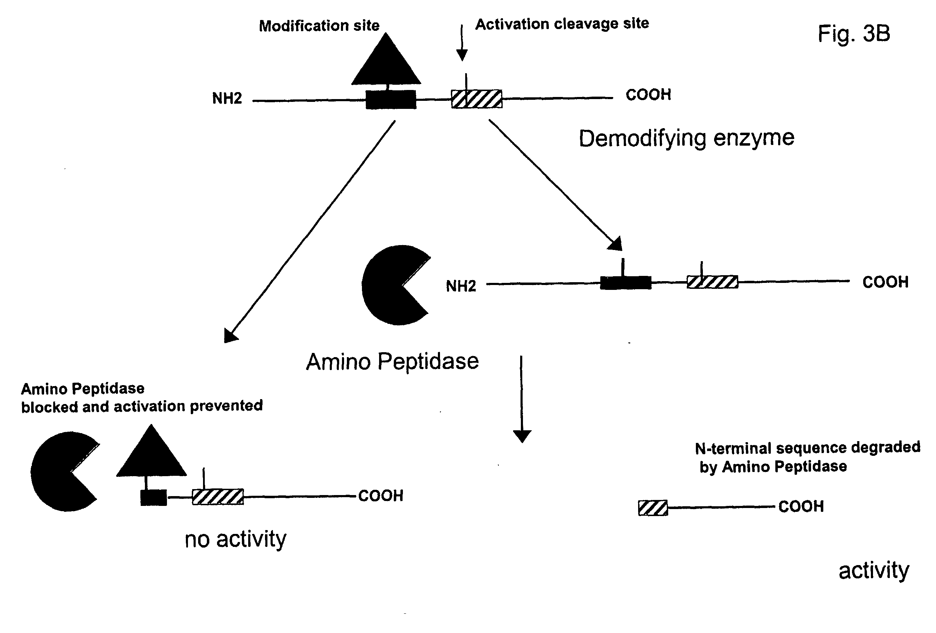

Fig. 1
   Schematic illustration of the structure of natural pro-urokinase. Natural pro-urokinase consist of a single polypeptide chain of 411 amino acid residues. The catalytic triad essential for activity is formed by His 204, Asp 245 and Ser 356, activation results from cleavage after Lys158 e.g. by plasmin its natural activator. The N-terminal part of the molecule from residue 1 to 158 is not required for activity.
Fig. 2
   Schematic representation of a detection enzyme construct for measuring protein (de)modification. The construct comprises overlapping modification target sequence and activation cleavage site. The modification target sequence (partially) overlaps with the activation cleavage sequence in such a way that the presence of the modification interferes with the action of the activating protease and thereby inhibits activation. This results in a signal decreasing with degree of modification if one starts with the unmodified form (A) or with a signal increasing with removal of modification if one starts with the modified form (B).
Fig. 3
   Schematic representation of a detection enzyme construct for measuring protein (de)modification comprising a modification target sequence N-terminal from the activation sequence. If the two sequences are not overlapping as in Fig. 2, one can employ an aminopeptidase for the activation step. The aminopeptidase removes amino acid residues from the N-terminus until the activation cleavage site is cleaved. The resulting conformational change leads to activity and prevents further action of the aminopeptidase. The presence of the modification interferes with activation and results in a lower activity if one starts with the unmodified form (A). If one starts with the modified form the signal will increase with increasing removal of modification (B).
Fig. 4
   Schematic representation of a detection enzyme construct for detection of modification with a signal increasing with modification. In this case the detection enzyme construct has an activation cleavage site and C-terminal a (partially) overlapping modification target sequence and an inactivation sequence. In unmodified form an inactivating protease cleaves the inactivation cleavage site and an inactive non-activatable form of the detection enzyme construct results. The presence of the modification interferes with inactivation enabling an activating protease to cleave the activation cleavage site and resulting in activity. Inactivating and activating protease can be the same but this is not necessary. Again one can start with the unmodified or modified form of the detection enzyme as described for Figures 2 and 3.
Fig. 5
   Schematic illustration of the structure of natural pro-caspases. Natural pro-caspases are single chain polypeptides consisting of a pro-domain, a large subunit, a linker and a small subunit. The critical step that leads to activation is a cleavage in the linker region, cleavage of the pro-domain seems to be less essential. The length of the linker region in the various procaspases varies from a single residue to over 20 residues. The figure is from Cohen GM, Biochem J (1997) 326, 1-16.
Fig. 6
   Schematic representation of a detection enzyme construct of the Caspase type with non overlapping activation cleavage site and modification target site. This approach can be used when no suitable overlapping activation cleavage site and modification target site can be constructed. A carboxypeptidase (A) or aminopeptidase (B) is used to inactivate the detection enzyme (construct) and the modification interferes with inactivation. Also in this case one can start with the modified or unmodified form as described above.
Fig. 7
   Schematic illustration of the structure of a pro-urokinase derived detection enzyme construct for protein kinase A or phosphatase activity. The natural sequence around the activation cleavage site of pro-urokinase was replaced by a sequence that is recognized and can be phosphorylated by protein kinase A. This site in non-phosphorylated form may be cleaved by MMP-14 whereas the phosphorylated form is not cleaved. In this way the presence of a phosphoryl group can be monitored by detecting the decrease of activity of the detection enzyme.
Fig. 8
   Effect of phosphorylation on activatability of detection enzyme UK_{PKA3} by MMP-14. The detection enzyme UK_{PKA3} was expressed and isolated as described. When the non-phosphorylated form was incubated with MMP-14 an active detection enzyme resulted that could be measured by conversion of the peptide substrate. After treatment with ATP and protein kinase K, the detection enzyme showed hardly any activity upon incubation with MMP-14.
Fig. 9
   Effect of protein kinase K treatment and phosphatase treatment on activation of detection enzyme UK_{PKA3}. The detection enzyme UK_{PKA3} was incubated with and without protein kinase A (PKA) and protein phosphatase (PP) and subsequently incubated with MMP-14. Thereafter activity was monitored by detection of pyro-Glu-Gly-Arg-pNA hydrolysis. Without kinase and phosphatase treatment (-PKA, -PP) the detection enzyme is activated, treatment with kinase (+PKA, -PP) results in a drop in activity, treatment with only phosphatase has hardly any effect (-PKA, +PP) and treatment with kinase followed by phosphatase (+PKA, +PP) restores activatability.
Fig. 10
   Dose response curve of protein kinase A effect on activatability of detection enzyme UK_{PKA3}. The detection enzyme UK_{PKA3} was incubated with various concentrations of protein kinase A. After incubation, activation with MMP-14 was performed and subsequently resulting activity was monitored by substrate hydrolysis. Increasing kinase amount leads to lower activatability.
Fig. 11
   Schematic illustration of the structure of a pro-urokinase derived detection enzyme construct for lysylhydroxylase activity. The natural sequence around the activation cleavage site of pro-urokinase was replaced by a collagenase-specific activation site (Arg Pro Leu Gly) and an overlapping lysyl hydroxylase modification site (Ile Lys Gly) and plasmin cleavage site (Lys Gly). The non-modified form is cleaved by plasmin as indicated resulting in an inactive form that cannot be activated by collagenase cleavage. The modified form cannot be cleaved by plasmin and can be activated by collagenase.
Fig. 12
   Effect of lysylhydroxylase activity on detection enzyme. Without plasmin treatment a slightly variable signal is observed not dependent on lysyl hydroxylase (grey bars). With plasmin treatment the signal was dependent on lysylhydroxylase concentration, an increasing signal with increasing concentration (black bars).
Fig. 13
   Dose response relationship between lysylhydroxylase concentration and activity after plasmin inactivation. The signal after plasmin inactivation i s linearly dependent on lysylhydroxylase concentration.
Fig. 14
   Schematic overview of reaction steps in lysylhydroxylase determination using pro-urokinase derived detection enzyme. The detection enzyme can be activated by collagenase leading to activity. Plasmin treatment results in cleavage at the inactivation site and leads to loss of activity/activatability. Modification by lysylhydroxylase of the indicated lysine residue (K) prevents inactivation by plasmin and results in a signal positively correlating with amount of modifying enzyme activity.
Fig. 15
   Schematic overview of construction and structure of a pro-caspase-3 based detection enzyme construct for measuring protein kinase A or protein phosphatase activity.
   (A) Two new restriction enzyme cleavage sites BamHI and EcoRI were constructed in a cDNA coding for wildtype pro caspase-3 by PCR.
   (B) The vector was cut with BamHI and EcoRI and subsequently an oligonucleotide pair coding for a kinase recognition and protease activation site was ligated into the gap.

   This vector codes for a caspase variant in which the very short linker in natural pro-caspase 3 was replaced by a longer linker containing a recognition sequence for protein kinase A overlapping with a cleavage recognition sequence of MMP-14.
Fig. 16
   Effect of protein kinase and protein phosphatase on pro-caspase-3 based detection enzyme construct. The light bars show the activity of the non-kinase treated detection enzyme construct without (left), with alkaline phosphatase (middle) or with phosphatase 2B (right) treatment. The dark bars show the activity of the protein kinase A treated detection enzyme construct without (left), with alkaline phosphatase (middle) or with phosphatase 2B (right) treatment.
Fig. 17
   Effect of protein kinase K treatment on activation of detection enzyme construct UK_{PKA3}. The detection enzyme construct UK_{PKA3} was incubated with and without protein kinase A (PKA) and then with the dipeptidyl aminopeptidase cathepsin C (CC). Activity was monitored by detection of pyro-Glu-Gly-Arg-pNA hydrolysis. Non-phosphorylated UK_{PKA3} (-PKA, -CC) and phosphorylated UK_{PKA3} (+PKA, -CC) both have little activity. CC can be used for activation, but activation of the phosphorylated detection enzyme construct (+PKA, +CC) results in much lower activity than activation of the non-phosphorylated detection enzyme construct (-PKA, +CC).

### Detailed description of the invention

The invention provides a method of determining a protein modifying activity in a sample, comprising incubating the sample with a substrate of said activity and determining the modification of said substrate, wherein said substrate is a detection enzyme construct containing a modification target sequence which is recognized and modified by said modifying activity. Said modification target sequence has in modified or non-modified form a different effect on activity or activatability of said detection enzyme construct. Apart from said modification target sequence said detection enzyme construct also contains a site cleavable by a protease that upon cleavage results in activation or in-activation of said detection enzyme construct. The difference in activity or activatability after cleavage by a protease may be determined by observing or measuring the resulting activity of the detection enzyme using a suitable substrate of the activated detection enzyme. The sample will usually be a biological sample, such as a biological fluid, a fraction of a biological fluid, a biological tissue, an extract of a biological tissue and a fraction of an extract of a biological tissue. The detection enzyme construct may be derived from a (pro) enzyme by providing it with a modification target sequence if not already present in its sequence and with one or more suitably positioned protease cleavage sites if not already present in its sequence.

The present invention also provides the detection enzyme construct per se, more particularly a modified (pro)enzyme derived from a (pro)enzyme by introduction therein of a suitably positioned modification target sequence if not already present and one or more suitably positioned protease cleavage sites if not already present.

The invention further provides a kit for determining a protein modifying or demodifying activity in a sample, comprising a detection enzym e construct as defined herein, together with at least one member from the group consisting of substrates for active detection enzyme, (in)activating proteases, buffer solutions, standard preparations, specific antibodies, microtiter plates and instructions for use; and provides a device for determining a protein modifying or demodifying activity in a sample, comprising a detection enzyme construct as defined herein.

The invention also provides DNA constructs coding for a suitable detection enzyme construct, wherein said DNA constructs can be used to modify cells or organisms in such a way that said cells or organisms express said detection enzyme construct; said modified cells or organisms expressing the detection enzyme construct can be used as a source for isolation of said detection enzyme construct in purified or partially purified form or can be used per se as parts of an in vitro or in vivo detection system.

The invention uses a detection enzyme construct containing a sequence that can be modified by a particular protein modifying activity resulting in a modified form of the detection enzyme. The non-modified form and modified form of the detection enzyme construct differ in their behaviour upon contact with a particular proteolytic enzyme resulting in a difference of enzyme activity. The resulting enzyme activity can be a measure of modification or absence of modification depending on the structure and sequence of the detection enzyme construct.

A large variety of detection enzyme constructs can be suitable for use in the invention as long as a proteolytic cleavage step is required for conversion of the pro-enzyme form of the detection enzyme construct to the active form or of the active form of the detection enzyme to an inactive form and a suitable detection system exists for detection and/or quantification of the resulting enzymatic activity. Particularly suitable as detection enzyme construct are detection enzymes derived from proteolytic enzymes since many proteolytic enzymes result from pro-enzymes activated by a proteolytic-cleavage, but also other pro-enzymes could be suitable as basis for such a detection enzyme construct. Within the group of proteolytic enzymes many possible candidates exist to form a basis for construction of a suitable detection enzyme construct. Particularly useful are pro-enzymes of the serine protease family (see Table II for a number of examples) or cysteine protease family (see Table III for a number of examples) since a lot of information is available on amino acid and DNA sequence, activity, activation-mechanisms and structure and because general detection methods for these enzymes are operational.

Within these families particularly pro-urokinase and pro-caspases are of interest since cleavage of pro-enzyme to enzyme leads to an enormous increase in activity (high zymogenicity), the resulting active enzymes are sufficiently specific to prevent unwanted cleavages, the resulting enzyme activity is easily detected using well known mature technology, and the proteins are stable and easily expressed in eukaryotic or bacterial expression systems.

As demonstrated in EP 0 691 409 B1 and European patent application No. 03 075 319.8, which have been mentioned above and are incorporated in their entirety herein by reference, these pro-enzymes may be modified by introduction therein of a new activation sequence. In those documents, such modification was required to allow specific measurement of the proteolytic activity recognizing said new activation sequence. Herein, replacement of the natural activation sequence by a new activation sequence is not an essential requirement, but it may be practical and will usually be preferable.

Apart from these elements, a detection enzyme construct of the invention should contain at least one site that can be modified by a particular protein modification process or activity, such as a protein modifying enzyme. The exact sequence of such a site mostly depends on the protein modifying activity or process one wants to detect or quantify. The position within the sequence of the detection enzyme construct is chosen in such a way that the presence or absence of the protein modification either has effect on enzymatic activity or activatability of the pro-enzyme.

A non-limited list of possible sequences that can be modified by a number of known protein modifying enzymes can be found in Table IV.

Apart from modifications around the activation site or modifications forming a target for protein modification, also other modifications in the sequence of the detection enzyme construct might be present. Such modifications include modifications increasing (thermal) stability, conferring resistance against proteases, conferring resistance against enzyme inhibitors, increasing the enzymatic activity, conferring reactivity to certain antibodies or ligands, aiding expression or purification, etc. In general such modifications are in parts of the detection enzyme construct not directly involved in the protein modification to be measured or the activation of the pro-enzyme construct to its active form. Another modification that might be useful is the introduction of an extra protease cleavage site N-terminal from the activation site. Cleavage at such a site would result in an inactive molecule that could be activated by an aminopeptidase. In some cases, an extra protease cleavage site C-terminal from the activation site could be appropriate, when a carboxypeptidase is used for activation. Deletion mutants lacking parts of the original sequence not needed for activity may be a suitable alternative. Such constructs may be preferable to achieve more rapid activation (or inactivation) of the detection enzyme construct by an aminopeptidase or carboxypeptidase.

Critical to the invention is a site that can be modified by the particular protein modifying activity one wants to detect or quantify. Such a site might be present naturally in the pro-enzyme construct, but more likely has to be introduced by adding or replacing a stretch of aminoacid residues expected or known to be a target for modification in proteins. The position of such a modification within the detection enzyme construct has to be chosen such that the absence or presence of the protein modification will interfere with activity or activation of the detection enzyme construct. Depending on the nature of the modification, the particular aminoacid sequence of the site to be modified, the detection enzyme and the protease used for activation or inactivation of the detection enzyme construct, a suitable position of such a modification target sequence should be determined. In general, but not necessarily, such modification target sequences are placed in the vicinity of the activation site.

Natural pro-urokinase occurs as a single chain polypeptide, cleavage by its natural activator plasmin after Lysine residue 158 leads to a molecule consisting of an N-terminal light chain (residues 1-158) and a C-terminal heavy chain (residues 159-411) connected by a single disulfide bridge. The two chain molecule is enzymatically active. It has been shown that residues 1-158 have no function in enzymatic activity, the isolated chain 159-411 has equal or almost equal enzymatic activity as the natural two chain urokinase. Further, it has been shown that the new N-terminus (I 159) in natural urokinase is very important for activity, cleavage at R 156 results in a C terminal chain starting with F 157 and no protease activity. Similarly cleavage at I 159 resulting in a C terminal chain starting with I 160 and no protease activity. So it appears that it is the newly formed N-terminus that is important for activity and not the way it is generated. It is possible to generate the new N-terminus with a single proteolytic cleavage at the right position with a regular protease or even by removing residues one-by-one with an amino peptidase. Activation with such an aminopeptidase is particularly effective when the number of aminoacid residues N-terminal to the activation sequence is limited. As soon as the I 159 becomes N-terminal, activity results. The inactive molecule starting with F 157 can be converted to an active molecule by removing F 157 and K 158 by an aminopeptidase.

Further research has shown that variations in the new N-terminal part of the heavy chain of cleaved pro-urokinase compatible with activity are very limited. Even replacing I 159 by a very similar A residue greatly affects activity. Modifications N-terminal from the natural cleavage position generally have no significant influence on activity but might result in pro-enzymes activatable by other proteins than the ones activating natural pro-urokinase. Examples of this have been described before in EP 0 691 409 B1. Also modifications in the aminoacid sequence in the heavy chain, more distant from the activation cleavage site are not necessarily deleterious for activity. Modifications around residue 180 have been described to lead to a molecule that in its two-chain form is an active protease resistant against its naturally occurring inhibitor PAI-1.

Pro-urokinase constructs suitable as detection enzyme in the present invention should contain a natural or modified C-terminal heavy chain and at least a short N-terminal chain connected by a peptide bond. The heavy chain should start with a sequence very similar to that of natural urokinase in order to retain enzymatic activity. The C- and N-terminal chains need not be connected by a disulfide bond like natural urokinase. The allowable length of the N-terminal chain may vary; it can be as short as 1 aminoacid residue or be as long or even longer than the N-terminal chain of natural urokinase. A sequence of residues immediately in front of the activation cleavage site determines which protease can activate the pro-urokinase construct to an active enzyme. A large variety of sequences has been shown to be compatible resulting in pro-urokinase constructs activatable by a variety of proteases other than plasmin, its natural activator. All such constructs could be employed in the invention and can have particular advantages for certain applications.

The above mentioned properties of pro-urokinase and actually of most of the members of the serine protease family and of the related cysteine protease family make these molecules particularly suitable to form the basis of a detection enzyme according to the present invention. In the following we use pro-urokinase as an example but the same principles hold for most of the members of its family.

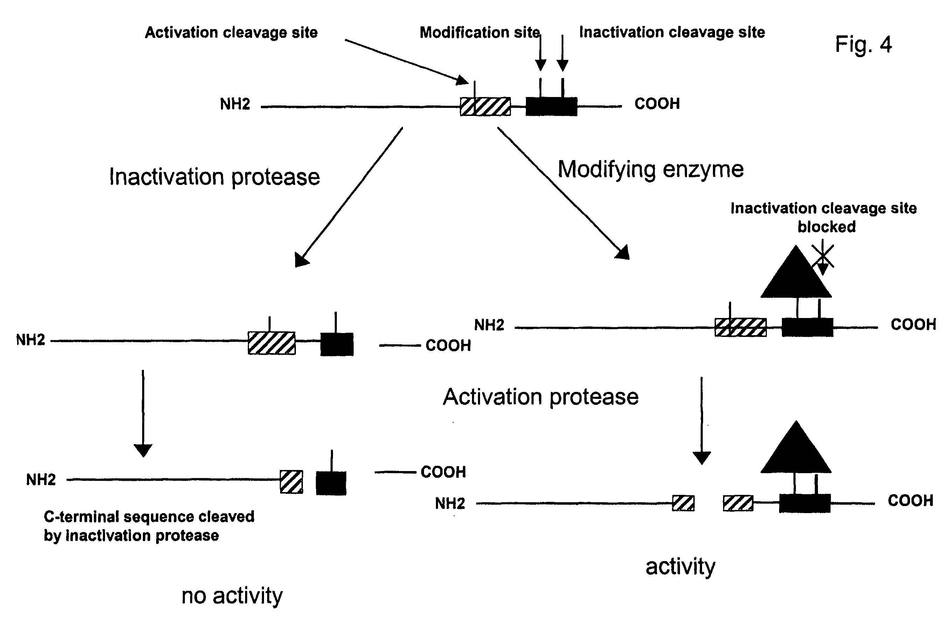

In the following part a number of specific embodiments of the invention based on the use of detection enzymes with urokinase like properties will be explained in more detail.
1. Modification target sequence (partially) overlaps with cleavage site for activating protease.
   In the case that the modification target site at least partly overlaps with the cleavage site for the activating protease a very simple and straightforward assay approach results, as illustrated in Fig. 2.
   One may start with the non-modified form of the pro-urokinase derived detection enzyme construct to be able to detect and quantify activities (most likely enzymatic activities but not necessarily limited to that) that lead to modifications of the target sequence (Fig. 2A) or start with the modified form of the detection enzyme to monitor removal of the modification already present (Fig 2B).
   Modification of the target sequence will also alter the recognition sequence for the activating protease (since they overlap) and will result in an altered activation behaviour. In most cases cleavage of the activation sequence will be inhibited by modification, thus resulting in lower activity of the detection enzyme. However, it is also conceivable that for particular combinations of modifications and activating proteases, modification will lead to increased ability of the detection enzyme construct to be cleaved and activated or result in higher activity. Both possibilities can be used in the method of the invention, although for particular applications one or the other of the possibilities may be preferred. The extent of modification of the activation sequence thus will be linearly related to the activity (or loss of activity) of the detection enzyme. So measurement of the activity of the detection enzyme will provide information on the activity of the modification process if one starts with the unmodified form of the detection enzyme or of the activity of the process removing the modification if one starts with the modified form of the detection enzyme.
2. Modification target sequence and activation cleavage sequence do not overlap.
   A slightly different approach can be followed when the modification target sequence and activating protease cleavage sequence are not sufficiently similar to overlap.
   A possible approach is illustrated in Fig. 3. Also in this case one may start with the unmodified form of the pro-urokinase derived detection enzyme to be able to detect or quantify activities leading to modification (Fig 3A) or start with the modified form of the detection enzyme to detect activities leading to removal of modification (Fig 3B).
   In this approach the modification target site is positioned N-terminal from the activation cleavage site. Activation is not induced by a regular (endo)protease cleaving the activation site, but brought about by an aminopeptidase that sequentially removes amino acid residues one-by-one or in steps of two aminoacid residues at a time from the N-terminus. When the aminopeptidase action finally cleaves the activation site, an active detection enzyme results. The presence of the modification in the modification target sequence, which is positioned between the N-terminus and the activation cleavage site, interferes with aminopeptidase action and would prevent activation (Fig 3A).
   If the sequence N-terminal from the activation cleavage site is long, the activation process might be inefficient or time consuming. In such cases it would be of advantage to use a detection enzyme with only a short N-terminal part. If this is not the case in the naturally occurring form of the detection enzyme one can accomplish this by either adapting the gene sequence of the detection enzyme or by removal of part of the N-terminal sequence by proteolytic cleavage at a site naturally occurring in the sequence of the detection enzyme or deliberately positioned in a suitable position.
   The possibilities described above are particularly suitable for detection and quantification of activities leading to removal of modifications, since in that case a signal linearly and positively related with the activity to be detected results. Removal of modification leads to more activation of the detection enzyme resulting in an increased signal. If this approach is used for detection or quantification of an activity leading to modification, a signal would result that decreases with increasing modification. Although such an inverse relationship between activity to be detected and signal actually measured might be acceptable, a positive relationship is much more desirable.
   To obtain a positive relationship between modification of the target sequence in the detection enzyme and the signal eventually generated by the active detection enzyme, a somewhat different construction of the pro-urokinase detection enzyme is required.
3. Modification affects inactivating cleavage
   In this approach the modification target sequence is positioned C-terminal of the activation cleavage site (Fig. 4) and should also contain, or suitably overlap,a protease sensitive bond. In the most simple form the protease required to hydrolyse the activation cleavage site would also cleave in the modification target site. Whereas cleavage of the activation site results in activity of the detection enzyme, cleavage of the site within the target sequence would lead to an inactive detection enzyme irrespective of cleavage at the activation site. The action of the modifying activity would interfere with cleavage in the modification target sequence and in that way prevent inactivation. The cleavage at the activation site leads to activation as expected, resulting in a signal positively related to modification. Also in this case one can start with the modified form to be able to detect demodifying activity analogous as described to the formats in Fig. 2 and Fig. 3.

Although all examples given above are especially suitable for pro-urokinase related detection enzymes, the approach is more general and would work for basically almost all pro-enzymes of the serine- and cysteine protease family with an activation mechanism based on cleavage of a peptide bond in a specific position and the release of a specific new N-terminus. Other examples of suitable pro-enzymes can be found in Table II and Table III.

Other pro-enzymes with a completely different activation mechanism than pro-urokinase and its family members are also suitable to be used as detection enzymes according to the invention. Particularly suitable are pro-caspases. In the following part a number of specific embodiments of the invention based on the use of detection enzymes with caspase like properties will be explained in more detail.

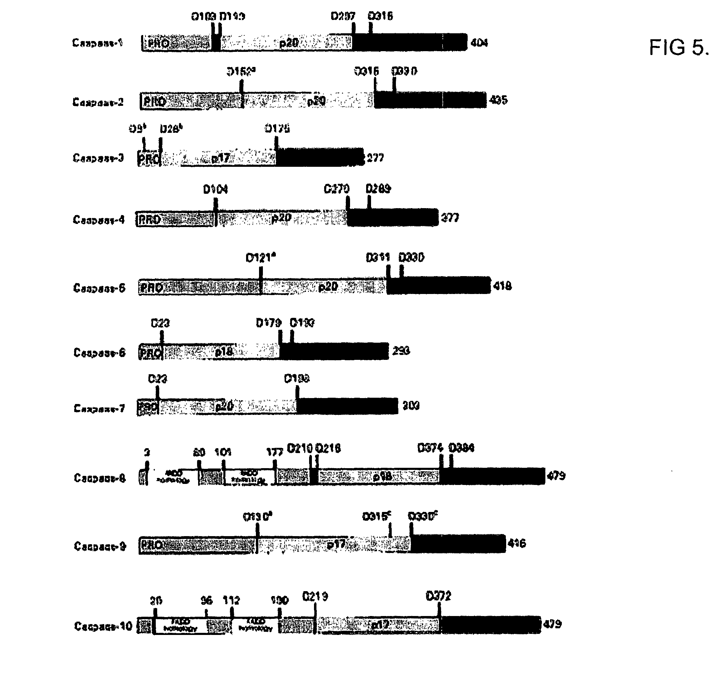

Pro-caspases are synthesized as single polypeptide chains. A proteolytic cleavage in a certain area of the peptide chain leads to a molecule consisting of two chains non-covalently attached to each other. The two chain forms of the caspases are enzymatically active (Fig 5).

In contrast to the activation of pro-urokinase and similar pro-enzymes, which require a proteolytic cleavage at a specific site and the release of a specific new N-terminus, activation of pro-caspases is not so stringent. Almost any cleavage in a certain area of the molecule is sufficient to result in activity. This property of pro-caspases has been employed in European patent application No. 03 075 319.8 to develop assay methods for a variety of proteolytic enzymes by introduction of a new cleavage sequence into the pro-caspase backbone. The length and sequence of this new cleavage sequence can be chosen with a considerable amount of freedom, giving much more flexibility than using pro-urokinase family members as a backbone.

These properties make pro-caspases very suitable to form the basis for detection enzymes according to the present invention.

In an analogous manner as described above for pro-urokinase, also pro-caspases can be adapted to be used as detection enzymes to detect or quantify protein modifications.

One possible approach is to construct a pro-caspase variant that has a sequence inserted in the activation area between the A and B chain sequences replacing the original activation sequence. This inserted sequence should contain a modification target sequence for the particular modification to be detected and a cleavage recognition sequence for a chosen activating protease.

As described for pro-urokinase derived detection enzymes, various possible approaches can be discerned.
1. Modification target sequence (partially) overlaps with cleavage site for activating protease.
   This approach is very similar to that described for pro-urokinase and schematically explained in Fig. 2. In most cases the unmodified target sequence will be cleaved by the activating protease resulting in caspase activity and the modified target sequence is protected against cleavage and activation. Again a detection enzyme according to this approach can be used to detect either the introduction of a modification or the removal of a modification. If one starts with the detection enzyme in its modified form, activities removing the modification will result in an increased signal, whereas if one starts with the unmodified form activities introducing the modification will result in a decreased signal. This approach will therefore be particularly suitable to detect the removal of modification, since that will lead to a positive relationship between signal and activity to be measured.
2. Modification target sequence and activation cleavage sequence do not overlap.

Two other approaches using pro-caspase based detection enzymes might be suitable for certain applications. In both cases a sequence containing a modification target site and an activation cleavage site will be introduced in the activation area. In these cases there does not need to be overlap in these two sequences.

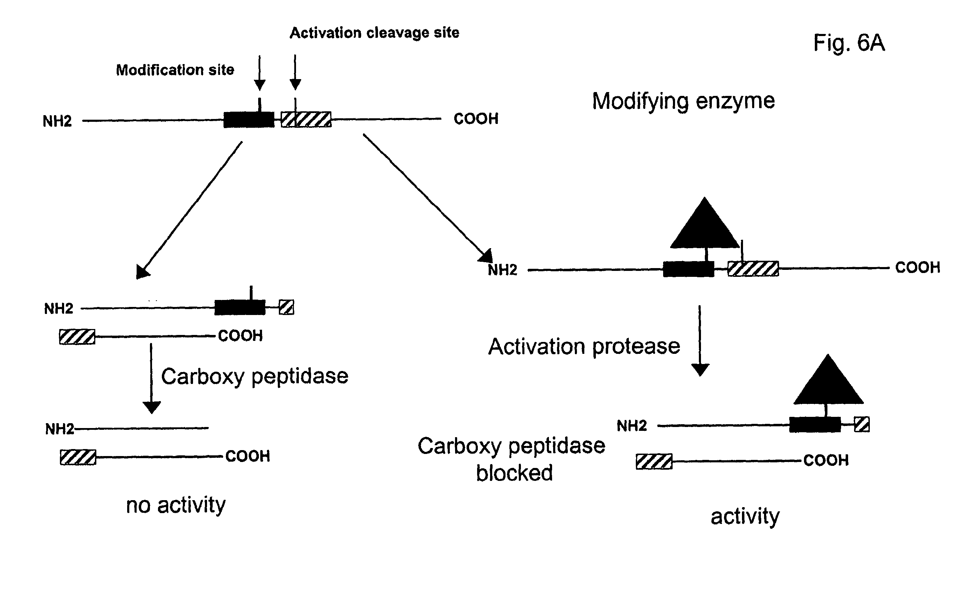

In one approach the modification target sequence is placed N-terminal from the activation cleavage site (Fig. 6A). Upon cleavage of the activation cleavage site, activity results. Subsequently a carboxypeptidase is added, an enzyme that sequentially removes one or more aminoacid residues from the newly formed C-terminus. The presence of the modification interferes with carboxypeptidase activity and will prevent carboxypeptidase to degrade residues in the A-chain critical for activity. We have discovered that the C-terminal residues of the A-chain are critical for caspase activity. In this way the presence of the modification will prevent inactivation and result in activity, whereas absence of the modification will allow carboxypeptidase to cut into the C-terminal part of the caspase A chain, removing the critical residues and lead to inactivation of the detection enzyme.

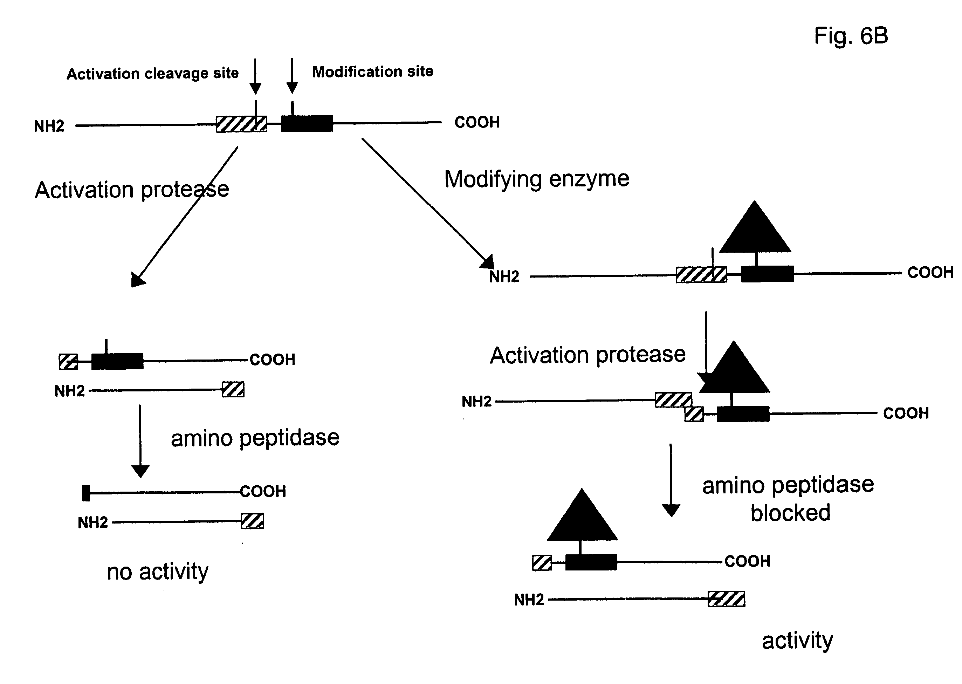

Another, very similar, approach requires an insertion with the modification target sequence C-terminal from the activation site (Fig. 6B). Cleaving the activation site will lead to activity. Subsequently an aminopeptidase is added that will cut into the N-terminal part of the caspase B chain again removing critical residues and leading to loss of activity. The presence of the modification prevents aminopeptidase to damage the B chain and will result in an active detection enzyme.

The activity leading to modification or removal of modification of a modification target site is most often caused by an enzymatic reaction of a specific enzyme. The activity of such an enzyme can be determined in a sample. Such a sample can consist of tissues, tissue extracts, cells, cell extracts, body fluids such as blood, plasma, urine, sputum etc., tissue- or cell-culture fluid, solutions of purified d'enzymes etc. All cells or tissues can be of plant, animal or human origin. The sample can also be of microbial origin (bacterial, yeast, fungus and the like or viral origin) or be taken from the medium on which the microogranism was cultured. The sample can also be taken from a nutrients. The sample can also consist of a purified or partially purified protein, or could be a food product. The sample can be any solid or liquid material in which one may detect or determine the presence or quantity of an activity leading to modification or removal of modification of a protein. In case the sample is taken from a solid it generally will be brought into solution by dissolving or suspending in a suitable liquid like water, buffer solutions, or organic solvents, detergents containing solutions etc.

The sample will be brought into contact with the detection enzyme with a suitable modification target sequence according to the invention under conditions suitable for the activity of the modification or demodification process. Conditions mean presence of other reactants; temperature; pressure; pH, reaction duration or any other parameter relevant for the activity of the process.

The presence and nature of other reactants depend on the specific modification or demodification activity to be determined. For determination of e.g. kinase activity, ATP will be required to provide the phosphoryl group to be transferred to the detection enzyme.

Metal ions like Mg²⁺ or Ca²⁺ might be required for the activity of certain modifying enzymes. Some enzymes might require co-factors for activity or reagents to stabilize their activity like sulphhydryl containing compounds such as dithiothreitol or dithioerythritol. For determination of e.g. lysylhydroxylase activity 2-oxoglutarate and oxygen are required as cofactor and donor for the hydroxyl group.

The conditions suitable for measurement of any modifying activity such as temperature, pressure, pH, reaction duration and other parameters are known to one skilled in the art.

In general one will employ temperatures in the range of 20°-37°C but lower temperatures from 0°C to 20°C or higher temperatures than 37°C can also be used. The pH will generally be in the neutral range e.g. from pH 6-pH 8, but certain activities or enzymes have a higher activity or stability at more basic conditions to pH values of pH 10 or occasionally even higher, other activities prefer more acid conditions to pH values as low as 3 or in special cases even lower. The duration of the modifying or demodifying step can vary in a wide range and depends i.e. on the activity to be determined and the required sensitivity and detection limit.

To determine the activity of the detection enzyme one can use a substrate for this enzyme. The use of synthetic instead of natural substrates might be preferred. Particularly suitable are substrates that on conversion by the active detection enzyme yield a coloured, fluorescent or luminescent signal. When the detection enzyme is a protease, synthetic peptide substrates with a color, fluorescence or luminescence generating label are suitable.

For urokinase, many such substrates are known and in general consist of a short peptide sequence coupled to a color or fluorescence generating label (Table V). Also for caspases such substrates are well known (Table VI). Also for many other detection enzyme systems such synthetic substrates are known to one skilled in the art. All enzyme systems with a suitable detection system can be used in the invention.

The detection system is not limited to the above mentioned substrates, many other detection systems such as those using natural substrates can be used. Ways of detection or quantification are known to those skilled in the art and would employ for example optical methods like turbidity and physical methods like HPLC, various forms of optical spectroscopy, mass spectroscopy etc.

Assays based on the invention can have various formats dependent on the properties of the modifying activity to be detected and the detection enzyme used. If modifying enzyme and detection enzyme can be active in the same condition (buffer, pH, temperature etc.) the complete set of reactions can be performed in one single step. If this is not the case it can be advantageous to perform the modification reaction and detection reaction subsequently. This allows separate optimization of the conditions in these steps. Especially if one wants to measure modifying activities in biological samples with many potential interfering components a rapid partial purification of the modifying enzyme can be of help. One very convenient way to do this is to use the so called immunocapture format. In this format one makes use of antibodies or other agents specifically binding the modifying enzyme to a surface of e. g. a microtiter plate. This approach enables rapid purification (capture) of one component from a complicated mixture. All such approaches are particular embodiments of the invention.

In another embodiment of the invention, one does not use the detection enzyme in purified or partially purified form, but one uses cells or organisms expressing or containing the detection enzyme. Expression can be such that the detection enzyme remains intracellularly, even in particular cellular compartments, bound to membranes or excreted into the medium. Such cells or organisms can be used as in vitro or in vivo detection systems suitable for measuring protein modifying activity within, bound to or in the immediate cellular surrounding or in the organism. This approach is particularly useful if one wants to measure the modifying activity in its natural surroundings with all interactions with other cellular components retained. The detection enzyme can be isolated from the cells and its modification be analysed as described before. Alternatively the detection enzyme activity can be determined directly on the cell surface or in lysed cells or a cell extract by adding the necessary components.

In this embodiment of the invention, detection is preferentially by fluorescence or luminescence although other detection principles can also be used.

A method of determination or quantification of a particular protein modification using a detection enzyme with a modification target sequence can be offered as a kit.

Typically, such a kit comprises containers with sufficient quantities of detection enzyme, a suitable substrate to detect the activity of the detection enzyme, a suitable standard preparation to quantify the modifying enzyme activity and (materials to prepare) buffer solutions. Furthermore, a kit might also contain one or more proteases useful for activation or inactivation of the detection enzyme, or specific antibodies recognizing the enzyme to be determined to increase the specificity of the assay by interfering with activity of that enzyme, by specifically capture the enzyme or by interfering with activity of other enzymes. A kit might also contain one or more assay plates in e.g. the regular 96-well, 384-well or 1536-well format. It might be advantageous to add stabilizers to the detection enzyme to increase its stability during transport and storage. Such stabilizers could be other proteins like albumin or gelatine, carbohydrates like mannitol, anti-oxidants, detergents, or other organic chemicals. Furthermore inorganic salts might also have beneficial effects on stability.

Most conveniently the detection enzyme is present in frozen solution or in lyophilized form that has to be reconstituted by adding buffer or water shortly before use. In addition, a detailed description how to perform the determination, preparing the samples and calculate the activity is included. A very convenient way to improve specificity as known in the art is to employ an immunocapture procedure: first the modifying enzyme to be measured is captured using a specific immobilized (monoclonal or polyclonal) antibody or other capturing device such as ligand, aptamer, etc. followed by measurement of the activity of the captured enzyme. Alternatively a kit or device might be of the "dipstick" type where all necessary reagents are present in a dry form immobilized on a strip or dot of material.

### Examples

The following examples are merely intended to illustrate the invention and in no way can be considered limiting.

### Example 1

### Preparation of a pro-urokinase derived detection enzyme for determination of protein kinase or protein phosphatase activity

### Construction of expression plasmid for pro-urokinase variants

The complete cDNA coding for human pro-urokinase (UKcol) with a collagenase specific cleavage site Arg Pro Leu Gly (RPLG in one-letter code) was cut from the expression plasmid pEV2UKcol (see EP 0691409 B1 for details) and cloned into the multiple cloning site of a new expression vector containing a neomycin resistance marker pcDNA3 (from Invitrogen).

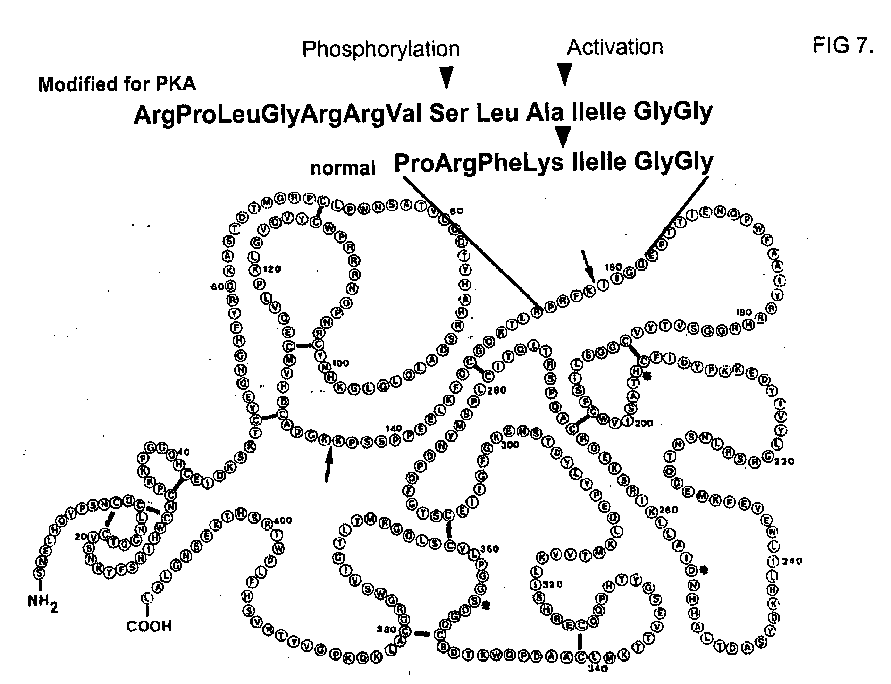

With polymerase chain reaction using primers 5'-ACC ATC GAt AAC CAG CCC TGG-3' (SEQ ID NO:1) and 5'-CCG CCT cga gGT CTT TTG GCC-3' (SEQ ID NO:2) (mutations indicated as lower case letters) two new unique restriction sites Cla*I* and Xho*I* were introduced flanking the region coding for the activation site. The resulting plasmid vector was used to construct pro-urokinase based detection enzymes by cutting the plasmid with ClaI and XhoI followed by insertion and ligation of two partially complementary oligonucleotides coding for the required mutated amino acid sequence. For construction of a detection enzyme with an overlapping protein kinase A phosphorylation and a collagenase activation sequence the following oligonucleotides were inserted in the ClaI XhoI cut plasmid: 5'-TCG AGG CGG CCG CTC GGG CGA AGA GTG TCC CTT GCC ATT ATT GGG GGA GAA TTC ACC ACC AT-3' (SEQ ID NO:3) and 5'-C GAT GGT GGT GAA TTC TCC CCC AAT AAT GGC AAG GGA CAC TCT TCG CCC GAG CGG CCG CC-3' (SEQ ID NO:4) resulting in a plasmid pcDNA3PKA3 coding for a pro-urokinase detection enzyme (UK_{PKA3}) with a sequence as shown in Fig. 7. This pro-urokinase variant contains an artificial site for modification by protein kinase A, Arg Val Ser Leu Ala (RVSLA in one-letter code) of which the Ser can be phosphorylated by PKA. This sequence overlaps with an artificial recognition sequence cleavable by various collagenases including matrix metallo protease -14 (MMP-14) Ser Leu Ala^Ile Ile Gly Gly. Collagenases can cleave at ^ between Ala and Ile. This cleavage leads to formation of an active urokinase variant.

### Expression of pro-urokinase based detection enzyme UK_{PKA3}

Cells (Chinese Hamster Ovary, CHO or Hamster Embryonic Kidney, HEK) were grown to 70-90% confluency in culture dishes. 1 µg of plasmid DNA pcDNA3PKA3 was mixed with medium and Fugene 6™ (Roche) and transfection was performed according to the manufacturer's instructions. To make stable transfectants, transfectants were selected by resistance to the neomycin analog G418. Transfected cells were cultured at various dilutions in medium containing selection agent G418 (1mg/ml) for 1-2 weeks. Remaining clones were selected, isolated with a pipette and cultured separately in medium containing selection agent. Production of pro-urokinase variants by these clones was tested by immunoassay (van Bohemen et al. Fibrinolysis 9 (1995) 343-349). Clones with high production levels were expanded and used for production, in culture disks, flasks, cell factories or any other suitable system known to one skilled in the art. For production, cells were routinely grown in triple flasks and conditioned culture medium was collected, centrifuged and stored at -20°C. The pro-urokinase based detection enzyme UK_{PKA3} was purified according to the methods described previously (JH Verheijen et al.Biochem J 323 (1997) 603-609).

### Example 2

### Determination of protein kinase activity and phosphatase activity

The modified pro-urokinase derived detection enzyme with a protein kinase A specific modification sequence UK_{PKA3} described in Example 1 was used in this example.

Microtiter plates (96-wells) were coated with a mouse monoclonal antibody recognizing human pro-urokinase and its modified forms. Coating was performed in phosphate buffered saline (PBS) overnight at 4 °C followed by washing with PBST (PBS with 0.01% (v/v) Tween 80).

Twenty microliters of culture medium from hamster embryonic kidney cells expressing the detection enzyme UK_{PKA3} and 80 µl PBST were pipetted into wells of the coated plate and incubated for 2 h at 37°C, followed by washing with PBST.

Phosphorylation of the detection enzyme UK_{PKA3} was performed as follows: 50 µl buffer A (40 mM Tris HCl pH 7.5, 20 mM MgCl₂, 0.1 mg/ml BSA), 25 µl cAMP dependent protein kinase catalytic subunit (PKA) (Promega, catalog number V5111) diluted in buffer A to 320 U/ml and 25 µl 0.2 mM ATP in buffer A were added and the plate was incubated during 7 h at 25°C or 37°C. After incubation the plate was washed with PBST.

De-phosphorylation was achieved by alkaline phosphatase (Sigma, catalog number P-2276) at 200 U/ml in 50 mM Tris HCl pH 9.0, 10 mM NaCl, 0.01 % (v/v) Tween 80 overnight at 37°C. After incubation the plate was washed with PBST.

Activation of the detection enzyme UK_{PKA3} by the collagenase MMP-14 (Chemicon, CC1043) at 24 ng/ml was done in 100 µl per well of 50 mM Tris HCl pH 7.6, 1.5 mM NaCl, 0.5 mM CaCl₂ and 1 µM ZnCl₂, 0.01% (v/v) BRIJ 35, 2 IU/ml heparin at 37°C.

Detection was performed by addition of 10 µl 6 mM pyro-Glu-Gly-Arg-pNA (S2444 Chromogenix) and incubation at 37°C. Color formation was monitored in a microtiterplate reader at 405 nm.

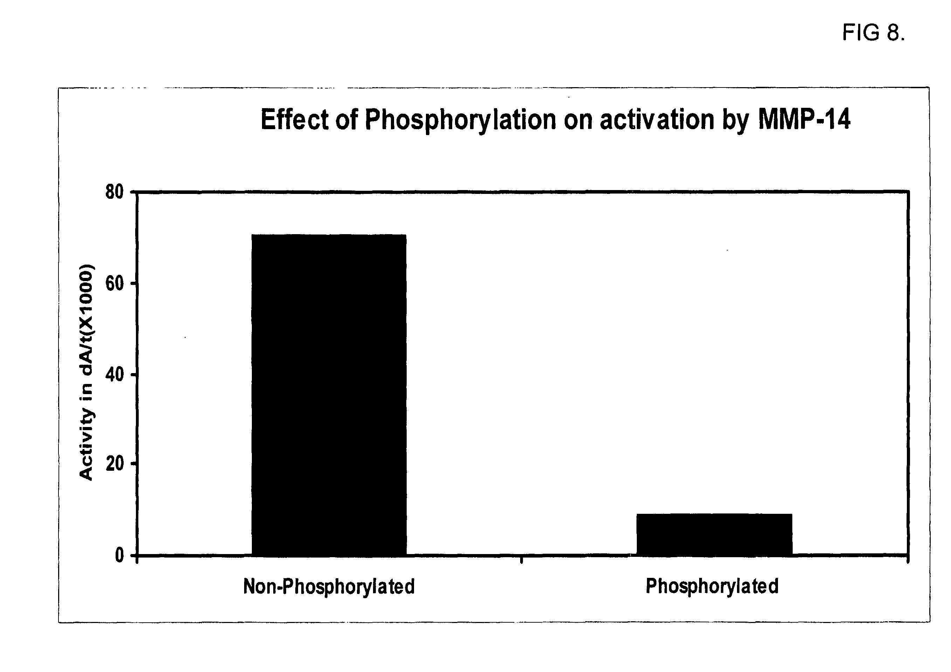

In Fig. 8 it is shown that the non-phosphorylated form of the detection enzyme UK_{PKA3} can be activated by MMP-14 resulting in color formation, whereas the phosphorylated form showed hardly any activity. This indicates that phosphorylation of the serine residue in the cleavage recognition sequence (see Fig. 7) interferes with activation.

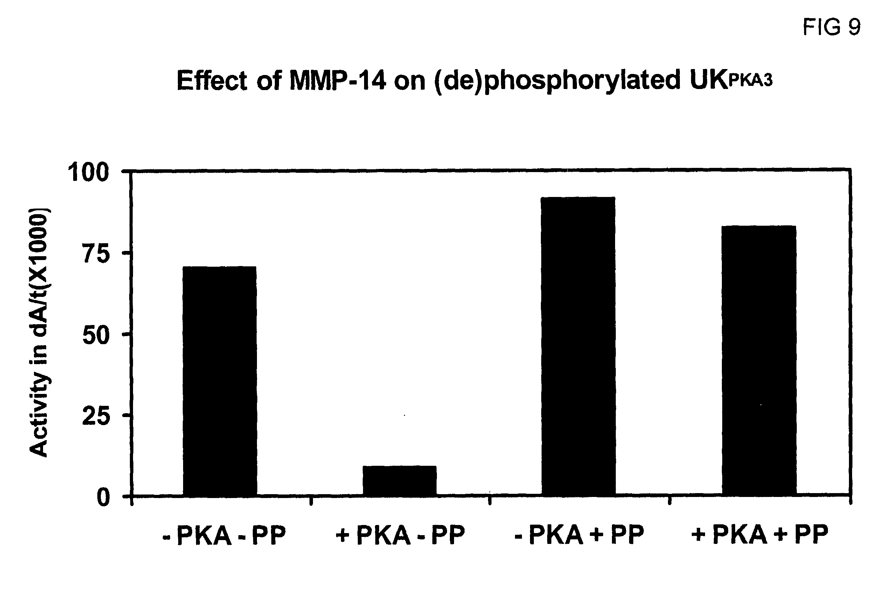

In Fig. 9 it can be seen that the non-phosphorylated form of the detection enzyme (first bar) is active, and that kinase treatment leads to loss of activity (second bar). Treatment of the non-phosphorylated form of the detection enzyme with alkaline phosphatase has no effect (third bar) and alkaline phosphatase treatment of the phosphorylated form leads to reappearance of activity (fourth bar).

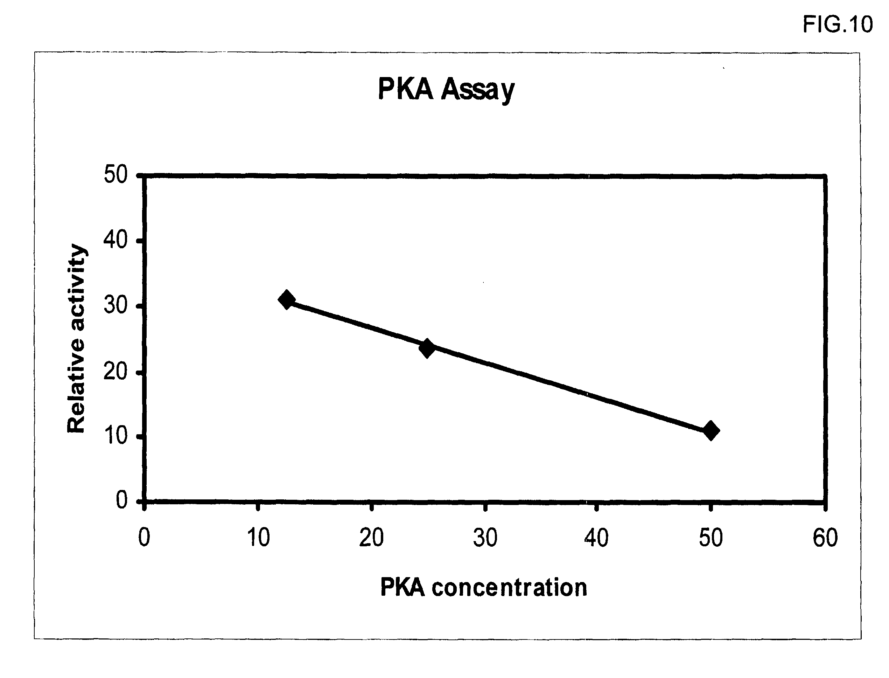

In Fig. 10 it is shown that the activity of the detection enzyme after MMP-14 cleavage decreases with PKA concentration indicating that increasing phosphorylation correlates with decreasing activity as expected in this approach (see also Fig. 2).

### Example 3

### Preparation of a pro-urokinase derived detection enzyme for determination of lysyl hydroxylase activity

### Preparation of detection enzyme

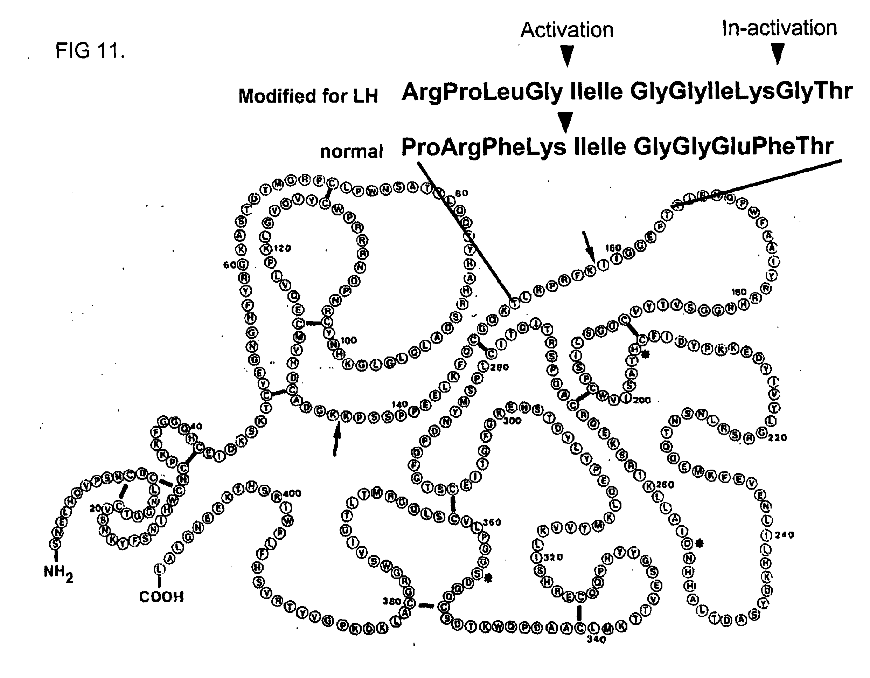

According to the procedure described in example 1, a modified pro-urokinase derived detection enzyme was prepared with a collagenase specific cleavage activation site, (Arg Pro Leu Gly, collagenase cleaves C terminal from Gly) and overlapping lysylhydroxylase recognition and plasmin inactivation sites (Ile Lys Gly, Lys can be hydroxylated and plasmin cleaves C terminal from Lys). (Fig. 11).

Oligonucleotides 5'-TCG AGG CGC CCC CTG GGC ATT ATT GGC GGG ATT AAG GGT ACC AT-3' (SEQ ID NO:5) and 5'- C GAT GGT ACC CTT AAT CCC GCC AAT AAT GCC CAG GGG GCG CC-3' (SEQ ID NO:6) were used for construction of the modification into the expression plasmid.

### Example 4

### Detection of lysylhydroxylase activity

Microtiterplates (96-well) were coated with a mouse monoclonal antibody recognizing human pro-urokinase and its modified forms. Coating was performed in phosphate buffered saline (PBS) overnight at 4°C followed by washing with PBST (PBS with 0.01% 1(v/v) Tween 80).

Twenty microliters of culture medium from CHO cells expressing detection enzyme and 80 µl of 50 mM TrisHCl pH 7.6, 1.5 mM NaCl, 0.5 mM CaCl₂, 1 µM ZnCl₂; 0.01% (v/v) BRIJ 35 (Buffer A) were pipetted to wells of the antibody coated microtiterplate and incubated overnight at 4°C, followed by washing with buffer A.

Subsequently 90 µl of 20 mM TrisHCl, 10 mM NaCl, 0.5 % (v/v) glycerol, 0.01 % (v/v) BRIJ 35 (buffer B) containing 1.12 mg/ml BSA, 82 µg/ml Catalase, 0.2 mM DTT, 1.1 mM ascorbic acid, 1.1 µM FeSO₄, 1.1 mM 2-oxo-glutarate) and 100 µl of a lysylhydroxylase preparation (lysylhydroxylase was expressed in HEK cells and purified as described in US2003/0219852A1) were added. After incubation for 3h or overnight at 30°C the plate was washed with buffer A. Hundred microliter of Na-periodate (5 mM in buffer B) was added and incubated at room temperature for 2h followed by washing with buffer A.

Bacterial collagenase 25 µg/ml in 100 µl buffer A was added and incubated for 1.5h at 37°C followed by washing with buffer A.

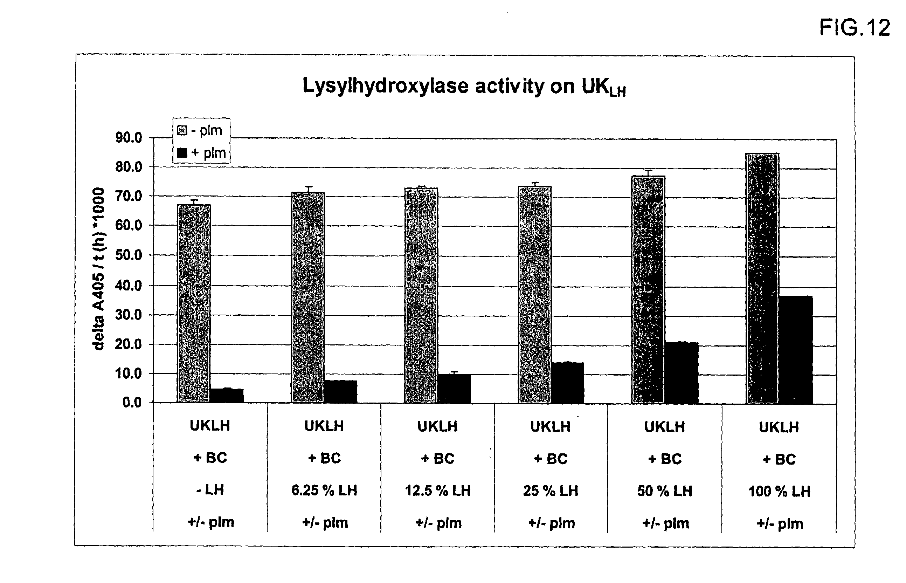

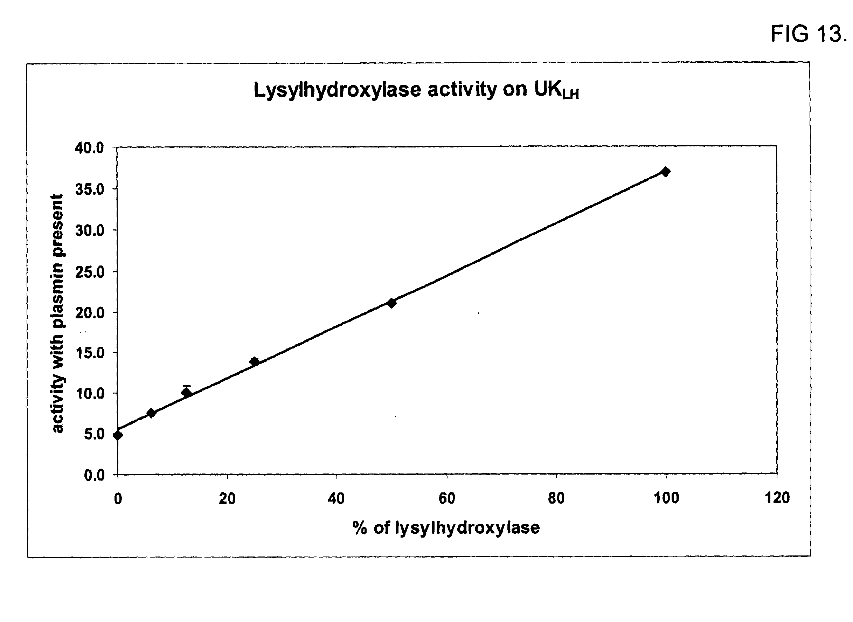

Hundred microliter of 50 nM plasmin in buffer A was added and incubated for 1h at 37°C followed by washing with buffer A. Trasylol solution 100 µl 50 KIE/ml in buffer A was added to inhibit remaining plasmin activity and incubated 30 min at 37°C followed by washing with buffer A. Chromogenic substrate 10 µl S2444 (pyro-Glu-Gly-Arg-pNA) 6 mM and 90 µl buffer A were added and incubated at 37°C. Color formation was monitored in a microtiter plate reader at 405 nm during 6 hours. The results are presented in Fig. 12. Without plasmin treatment (grey bars) a slightly variable signal was observed showing no dependence on lysylhydroxylase concentration. With plasmin treatment the signal was clearly dependent on lysylhydroxylase concentration (Fig. 12, Fig. 13).

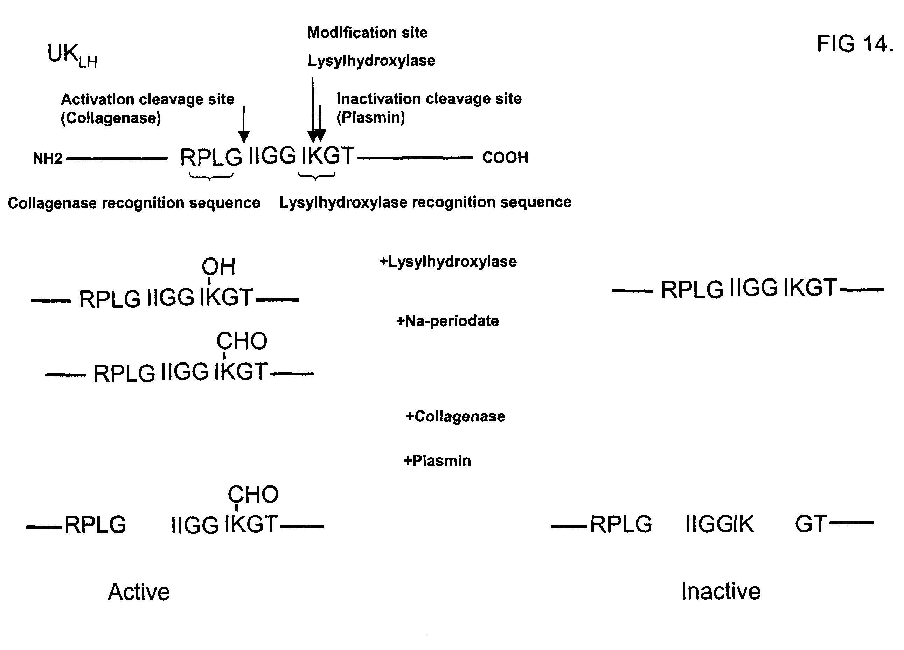

The various reaction steps in the determination are schematically summarized in Fig. 14.

The detection enzyme can be activated by collagenase, leading to activity. Plasmin treatment results in cleavage at the inactivation site and leads to loss of activity.

Modification of the overlapping plasmin cleavage site and modification site by lysylhydroxylase (partially) prevents inactivation by plasmin resulting in a signal positively correlating with amount of modifying enzyme activity.

### Example 5

### Preparation of pro-caspase derived detection enzyme for protein kinase A or protein phosphatase activity

### Preparation of c-DNA coding for detection enzyme

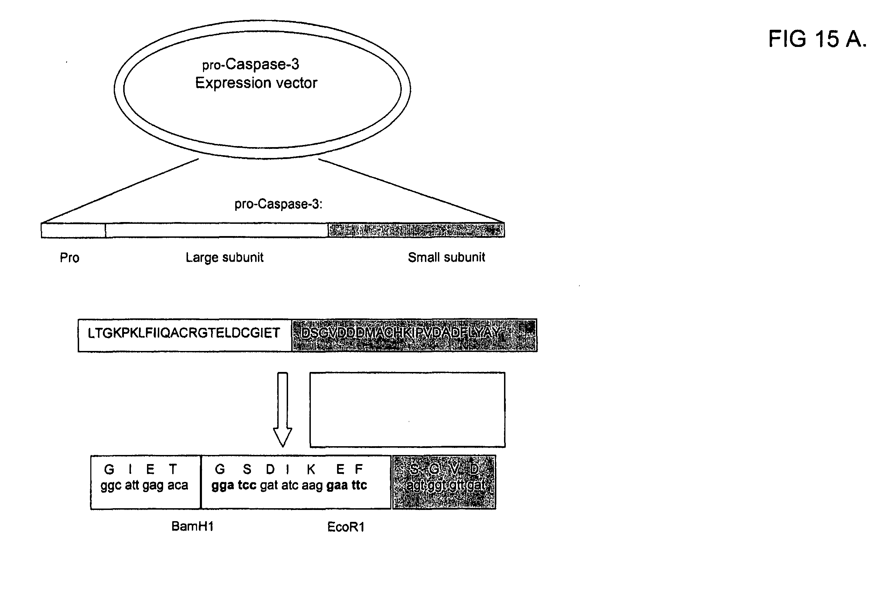

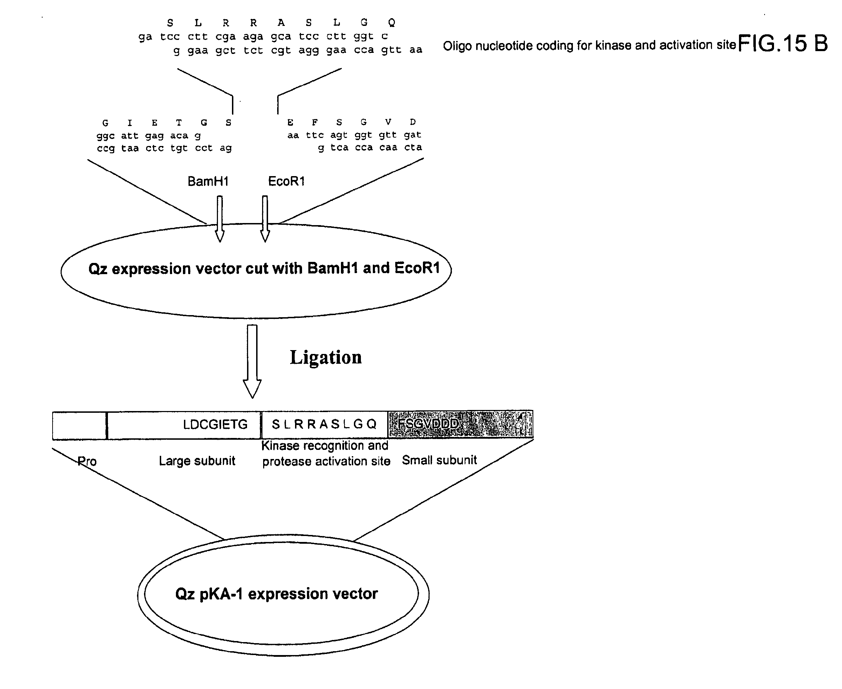

A cDNA coding for pro-caspase-3 cloned into an expression vector having a sequence coding for a His-tag enabling rapid purification using Ni-chelate chromatography and regulatory sequences enabling expression in *E.coli* was obtained from the ATCC (number MGC-21241). This plasmid was used as a basis for construction of similar plasmids coding for pro-caspase-3 based detection enzymes. To enable rapid construction of variants two new silent restriction sites (BamHI and EcoRI) were introduced by PCR using oligonucleotides 5'-GGA TCC TGT CTC AAT GCC ACA GTC CAG-3' (SEQ ID NO:7) and 5'-GAT ATC AAG GAA TTC AGT GGT GTT GAT GAT GAC ATG GCG-3' (SEQ ID NO:8) as primers (Fig. 15). After digestion with BamHI and EcoRI a novel coding sequence was introduced using two oligonucleotides. For preparation of QZ_{PKA}, a detection enzyme for protein kinase A activity, the following sequence was introduced with oligonucleotides 5'-GA TCC CTT CGA AGA GCA TCC CTT GGT C-3' (SEQ ID NO:9) and 5'-AA TTG ACC AAG GGA TGC TCT TCG AAG G-3' (SEQ ID NO:10). The resulting plasmid was used to transform *E.coli* (Rosetta DE3 pLys Novagen). Cultures were grown to an O.D. of 0.45-0.7 cooled to 30°C and induced with 1 mM IPTG. After shaking at 30°C, cells were centrifuged for 10 min, at approximately 5000 xg at 4°C and either immediately used or stored frozen at -20°C. Bacterial pellets were mixed with lysis buffer (50 mM Tris HCl pH 8.0, 300 mM NaCl, 10 mM imidazol) and Bugbuster™ (Novagen), incubated at 4°C while stirring for 30 min and centrifuged at ca. 5000 x g for 10 min at 4°C. The supernatant was brought to a nickel Sepharose column (Amersham Biosciences) (2 ml column volume for 30-50 ml original culture volume) and subsequently washed with 20 volumes of 50 mM Tris HCl pH 8.0, 300 mM NaCl, 20 mM imidazole (wash buffer) and eluted with elution buffer (wash buffer with 250 mM imidazole instead of 20 mM). The peak-fractions were desalted using a PD-10 column (Amersham Bioscience) equilibrated with 10 mM Tris HCl pH 7.0, 1.5 mM NaCl, 0.01 (v/v) BRIJ 35 buffer.

### Example 6

### Determination of protein kinase or protein phosphatase activity using modified pro-caspase

The pro-caspase-3 derived protein kinase A substrate (QZ_{PKA1}) produced as described in example 5 was used in this experiment.

Microtiter plates (96 wells) were coated with a 5 µg/ml solution of anti caspase-3 rabbit IgG in phosphate buffered saline (PBS) during 1 h at 37°C and subsequently washed with PBST. Purified QZ_{PKA1} 100 µl in 50 mM HEPES pH 7.4, 100 mM NaCl, 1 mM EDTA, 0.1% (w/v) CHAPS, 0.1% (w/v) caseine, 0.05% (v/v) Triton-X-100 (capture buffer) was pipetted to wells of the coated microtiterplate and incubated for 2 h at 37°C followed by washing in PBST.

Dephosphorylation was performed either with alkaline phosphatase (Sigma, catalog number P-2276) at 200 U/ml in buffer A (40 mM TrisHCl pH 7.5, 20 mM Mg Cl₂, 0.1 mg/ml bovine serum albumin overnight at 37°C or with phosphatase 2 B (Calbiochem catalog number 539568) at 1.5 microgram/ml in buffer A in the presence of calmodulin at 37°C overnight, followed by washing with PBST.

Phosphorylation of the captured detection enzyme was performed as follows: to each well 50 µl of buffer A (40 mM Tris HCl pH 7.5, 20 mM MgCl₂, 0.1 mg/ml BSA), 25 µl PKA (Promega, catalog number V5111) diluted in buffer A to 320 U/ml and 25 µl ATP 0.2 mM in buffer A were added and the plate was shaken for 20 seconds and incubated for 7 h at 37°C and washed with PBST.

Activation by MMP-14 was done as follows: 80 µl of MMP-14 (Chemicon CC1043) at 24 ng/ml in 50 mM Tris HCl pH 7.6, 1.5 mM NaCl, 0.5 mM CaCl₂ and 1 µM ZnCl₂, 0.01% (v/v) BRIJ 35, 2 IU/ml heparin was added to the wells and incubated for 3 h at 37°C.

Activity was measured after addition of 10 µl 8 mM Asp-Glu-Val-Asp-pNA and 10 µl 100 mM DTT and color formation was followed at 405 nm with a plate reader.

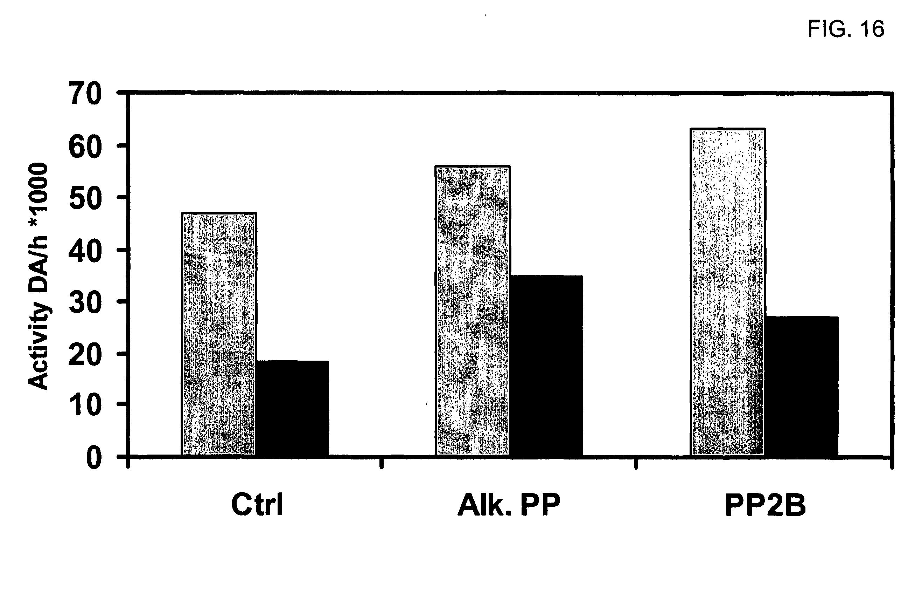

Fig. 16 shows the result of the experiment. The light bars show the activity of the non-kinase treated QZ_{PKA1} without treatment (left), with alkaline phosphatase treatment (middle), or with phosphatase 2B treatment (right). The results indicate that most of the QZ_{PKA1} is produced in the non-phosphorylated form and the effect of phosphatase treatment is small. Treatment with protein kinase A in the presence of ATP (dark bars) results in a significant drop in activity explainable by partial phosphorylation.

A schematic representation of the protocol can be found in Fig. 2A.

### Example 7

### Determination of protein kinase activity using an aminopeptidase as activation enzyme

The modified pro-urokinase derived detection enzyme with a protein kinase A specific modification sequence UK_{PKA3} described in Example 1 was used in this example.

Microtiter plates (96-wells) were coated with a mouse monoclonal antibody recognizing human pro-urokinase and its modified forms. Coating was performed in phosphate buffered saline (PBS) overnight at 4 °C followed by washing with PBST (PBS with 0.01% (v/v) Tween 80).

Hundred microliters of culture medium from hamster embryonic kidney cells expressing the detection enzyme UK_{PKA3} were pipetted into wells of the coated plate and incubated for 2 h at 37°C, followed by washing with PBST.

Phosphorylation of the detection enzyme UK_{PKA3} was performed as follows: 50 µl buffer A (40 mM Tris HCl pH 7.5, 20 mM MgCl₂, 0.1 mg/ml BSA), 25 µl cAMP dependent protein kinase catalytic subunit (PKA) (Promega, catalog number V5111) diluted in buffer A to approximately 300 U/ml, 25 µl 0.2 mM ATP in buffer A were added and the plate was incubated overnight at 37°C. After incubation the plate was washed with PBST.

Activation with the dipeptidyl aminopeptidase cathepsin C was performed in 100 µl cathepsin C buffer (50 mM Na-Phosphate pH 6.0, 100 mM NaCl, 2 mM EDTA, 10 mM L-cysteine, 0.1 % (w/v) bovine serum albumin, 0.01% (v/v) Tween 80 containing 3.3 µg/ml cathepsin C (Sigma D 7052) overnight at 37°C, followed by washing with PBST.

Detection was performed by addition of 90 µl assay buffer (50 mM Tris HCl pH 7.6, 1.5 mM NaCl, 0.5 mM CaCl₂ and 1 µM ZnCl₂, 0.01% (v/v) BRIJ 35), and 10 µl 6 mM pyro-Glu-Gly-Arg-pNA (S2444 Chromogenix) and incubated at 37°C. Color formation was monitored in a microtiterplate reader at 405 nm.

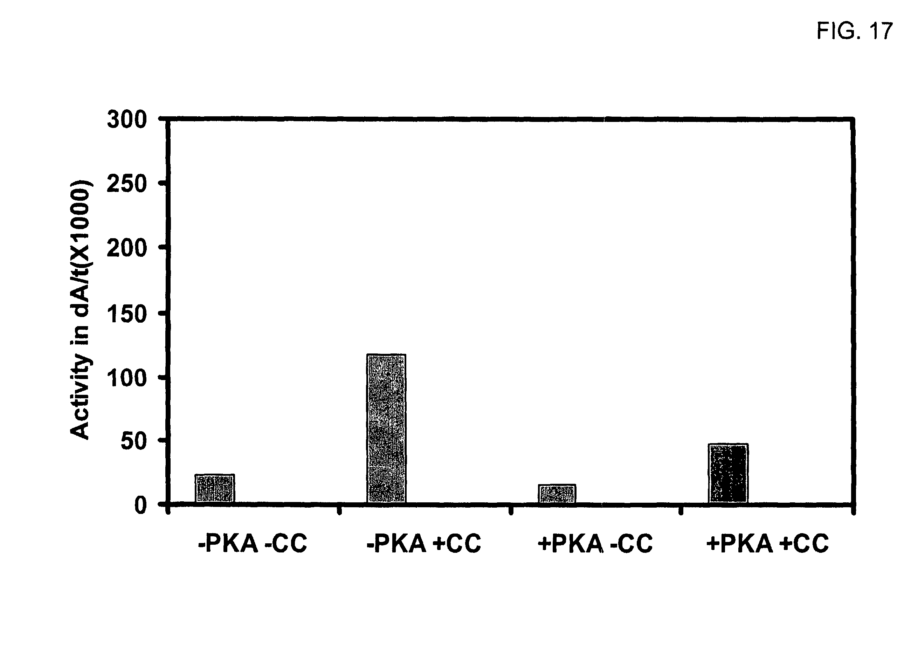

In Fig. 17 it is shown that the non-phosphorylated (-PKA-CC dark bar) form of the detection enzyme UK_{PKA3} has hardly any activity, but can be activated by the aminopeptidase cathepsin C resulting in an increase of activity (-PKA+CC light bar). If the detection enzyme is phosphorylated by protein kinase A, activity resulting after cathepsin C treatment is much lower (compare +PKA-CC with +PKA-CC).

**Table I**

| Non limited list of protein modifications, target amino acid residues and activities leading to or removing modification. | | | |
|---|---|---|---|
| Modification | Target residue | Enzyme modifying activity | Enzyme demodifying activity |
| Phosphorylation | Tyr, Ser, Thr, His | Protein-kinases, phosphorylases | Protein phosphatases |
| Glycosylation | Asn, Ser, Thr, Hyp | Glycosyltransferases | Not known |
| Hydroxylation | Pro, Lys | Hydroxylases | Not known |
| Methylation | Lys | Protein methyl transferase | Not known |
| Acetylation | Lys, N-terminus | Protein acetyl transferase | Not known |
| Formylation | N-terminal Met | i.a. Introduced during synthesis | Polypeptide deformylase |
| Carboxylation | Glu | Gamma glutamyl carboxylase | Not known |
| Prenylation | Cys | Prenyltransferase | Not known |
| Myristylation | Cys, His | Not known | Not known |
| Oxidation | Met | Non-enzymatic | Not known |
| Glycation | Lys | Non-enzymatic | Not known |
| Disulfide formation | Cys | Enzymatic/non-enzymatic | Protein disulfide isomerase |

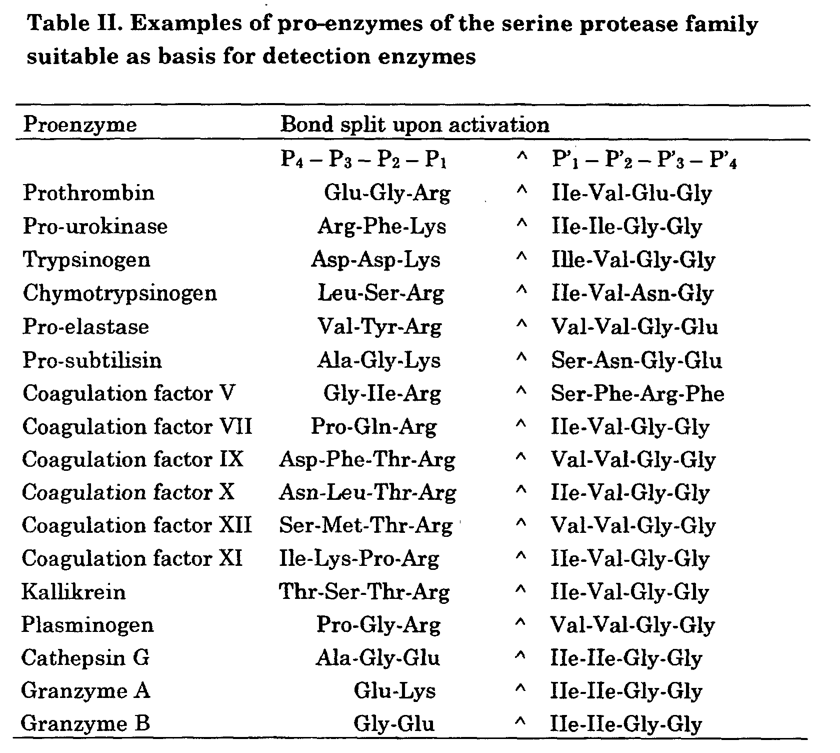

Sequences obtained from SWISS-PROT, GenBank or PIR databases.

**Table III**

| **Examples of proenzymes of the cysteine protease family suitable as basis for detection enzymes.** | |
|---|---|
| Pro-Caspase | -1 |
| Pro-Caspase | -2 |
| Pro-Caspase | -3 |
| Pro-Caspase | -4 |
| Pro-Caspase | -5 |
| Pro-Caspase | -6 |
| Pro-Caspase | -7 |
| Pro-Caspase | -8 |
| Pro-Caspase | -9 |
| Pro-Papain | |
| Pro-cathepsin B | |
| Pro-cathepsin L | |
| Pro-cathepsin K | |

**Table IV**

| **Recognition Motifs of Protein Kinases** | | |
|---|---|---|
| Protein Kinase | Abbreviation(s) | Recognition Motifs |
| | | |
| Glycogen synthase kinase | GSK-3 | S*-X-X-X-S(P) |
| Calmodulin dependent protein kinase II | CaMK II | R-X-X-S/T* R-X-X- S/T*-V |
| | CdC2 | S/T*-P-X-R/K |
| | MAPK, Erk | P-X-S/T*-P |
| | CGPK | R/K-X-S/T* |
| | PKA, cAMPK | R-X-S/T* R-R/K-X-S/T* |
| Caseine Kinase I | CK-1 | S(P)-X-X-S/T* |
| Caseine Kinase II | CK-2 | S/T*-X-X-E |
| | PhK | K/R-X-X-S*-V/I |
| | PKC | S/T*-X-K/R K/R-X-X-S/T* K/R-X-S/T* |
| | AbI | I/V/L-Y*-X-X-P/F |
| | EGF-RK | E/D-Y*-X E/D-Y*-I/L/V |
| | | |
| | | |
| | | |
| | | |

Davis RJ, J Biol Chem 268 (1993) 14553-14556
Pearson RB , Kemp BE, Methods in Enzymology 200 (1991) 62-81
Flotow H et al. J Biol Chem 265 (1990) 14264-14269
Fiol CL et al. J Biol Chem 265 (1990) 6061-6065
Russo GL et al. J Biol Chem 267 (1992) 20317-20325
Roach PJ J Biol Chem 266 (1991) 14139-14142

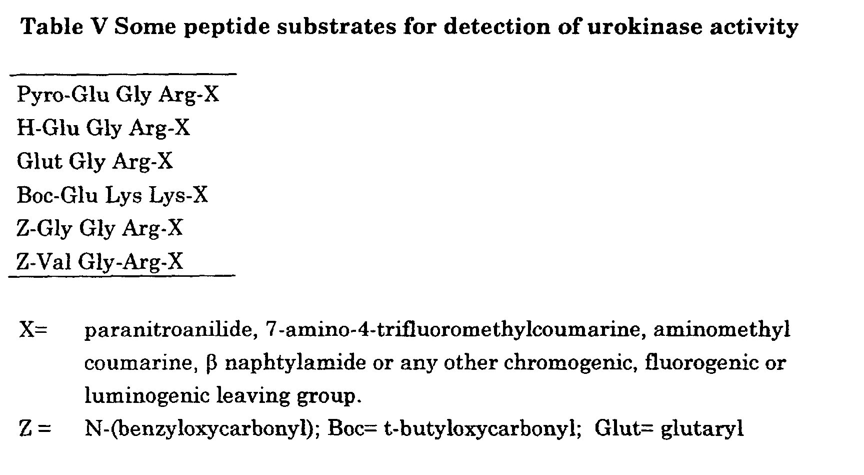

**Table VI**

| **Peptide substrates for detection of caspase activity** | |
|---|---|
| Substrate | Preferred caspase |
| VAD-X | 1 |
| DEVD-X | 3, 6, 7, 8 |
| VEID-X | 6,8 |
| IETD-X | 8, 9, 10 |
| WEHD-X | 1, 4, 5 |
| YVAD-X | 1, 4, 5 |
| VDVAD-X | 2 |
| X can be -pNA (para-nitro-anilide), -AFC (7-amino-4 trifluoro methyl coumarine), -AMC (amino methyl coumarine), or any other chromogenic or fluorogenic leaving group. | |

## Claims

1. A method for determining a protein modifying activity or protein demodifying activity in a sample comprising
providing a detection enzyme construct comprising at least one site of modification and at least one site of activation or inactivation, wherein activation or inactivation of the detection enzyme construct results from cleavage of said site of activation or inactivation by a protease, wherein said site of modification of the detection enzyme construct is capable of being modified by the protein modifying activity to be determined or demodified by the protein demodifying activity to be determined, and wherein modification or demodification of said site of modification affects cleavage of the said site of activation or inactivation by the said protease,
contacting the sample with the said detection enzyme construct under suitable conditions for the protein modifying or demodifying activity to be determined,
contacting the detection enzyme construct with the said protease under suitable conditions for activity of the said protease, and
determining the occurrence or level of detection enzyme activity.

2. A method according to claim 1, wherein said detection enzyme construct comprises a site of modification and a site of activation, wherein activation of the detection enzyme construct results from cleavage of said site of activation by an activation protease to form active detection enzyme, wherein said site of modification of the detection enzyme construct is capable of being modified by the protein modifying activity to be determined or demodified by the protein demodifying activity to be determined, and wherein modification or demodification of said site of modification determines whether said activation protease can cleave the said site of activation, and the method comprises contacting the detection enzyme construct with the said activation protease under suitable conditions for activity of the said activation protease.

3. A method according to claim 1, wherein said detection enzyme construct comprises a site of modification, a site of activation and a site of inactivation, wherein activation of the detection enzyme construct results from cleavage of said site of activation by an activation protease to form active detection enzyme, and inactivation of the detection enzyme construct or detection enzyme results from cleavage of said site of inactivation by a deactivation protease, wherein said site of modification of the detection enzyme construct is capable of being modified by the protein modifying activity to be determined or demodified by the protein demodifying activity to be determined, and wherein modification or demodification of said site of modification determines whether said activation protease can cleave the said site of activation or said inactivation protease can cleave the said site of inactivation, and the method comprises contacting the detection enzyme construct with the said activation protease under suitable conditions for activity of the said activation protease, and before, after, or simultaneously therewith, contacting the detection enzyme construct or detection enzyme with the said inactivation protease under suitable conditions for activity of the said inactivation protease.

4. A method according to claim 3, wherein modification or demodification of said site of modification determines whether said inactivation protease can cleave the said site of inactivation.

5. A method according to any one of claims 1-4, wherein active detection enzyme is detectable by its conversion of a substrate of the detection enzyme to a reaction product and detection enzyme activity is determined by contacting the detection enzyme construct or detection enzyme with said substrate of the detection enzyme and determining the occurrence or level of conversion of said substrate.

6. A method according to any one of claims 1-5, wherein said detection enzyme construct is derived from a protease enzyme or protease pro-enzyme.

7. A method according to claim 6, wherein said detection enzyme construct is derived from a serine protease enzyme or serine protease pro-enzyme or from a cysteine protease enzyme or cysteine protease pro-enzyme.

8. A method according to any one of claims 1-5, wherein said detection enzyme construct is derived from a pro-enzyme selected from the group consisting of pro-urokinase, pro-granzyme, pro-caspase-3 and pro-caspase-7.

9. A method according to any one of claims 1-5, wherein said detection enzyme construct is derived from pro-urokinase.

10. A method according to any one of claims 1-5, wherein said detection enzyme construct is derived from pro-caspase-3.

11. A method according to any one of the previous claims, wherein said detection enzyme construct contains an artificial modification site, or an artificial activation cleavage site, or an artificial inactivation cleavage site, or any combination thereof, and optionally further changes, introduced into a protease enzyme or protease pro-enzyme in such a way that modification or demodification of said modification site affects activatability of the detection enzyme construct by treatment with said activation protease or inactivatability of the detection enzyme construct or detection enzyme by treatment with said inactivation protease.

12. A method according to claim 11, wherein the activation protease is selected from the group consisting of (a) the natural activation protease of said protease pro-enzyme, (b) a protease specifically cleaving an artificial activation cleavage site introduced into said protease pro-enzyme, (c) an aminopeptidase, and (d) a carboxypeptidase.

13. A method according to claim 11, wherein the inactivation protease is the same or different from the activation protease and is selected from the group consisting of (a) a natural inactivation protease of said protease enzyme, (b) a protease specifically cleaving an artificial inactivation cleavage site introduced into said protease pro-enzyme, (c) an aminopeptidase, and (d) a carboxypeptidase.

14. A method according to any one of the previous claims, wherein said protein modifying activity or protein demodifying activity is an enzymatic activity.

15. A method according to claim 14, wherein said enzymatic activity is selected from the group consisting of of protein kinase activity, protein phosphatase activity, protein hydroxylase activity, protein glycosyltransferase activity or protein prenyltransferase activity.

16. A method according to any one of the previous claims for determining a protein demodifying activity, wherein the sample is contacted with pre-modified detection enzyme construct.

17. A method according to any one of the previous claims, wherein the sample is contacted with a purified or partially purified detection enzyme construct, or with a cell expressing or containing the detection enzyme construct, or with the content of such a cell or of a specific compartment of such a cell, or with the culture medium of such a cell.

18. A method according to claim 17, wherein the sample is contacted with a cell expressing or containing the detection enzyme construct and then either the cell as a whole, or the content of the cell or of a specific compartment of the cell, or the culture medium of the cell is contacted with the activation and/or inactivation protease and detection enzyme substrate.

19. A detection enzyme construct useful for determining a protein modifying activity or protein demodifying activity in a sample, comprising at least one site of modification and at least one site of activation or inactivation, wherein activation or inactivation of the detection enzyme construct results from cleavage of said site of activation or inactivation by a protease, wherein said site of modification of the detection enzyme construct is capable of being modified by the protein modifying activity to be determined or demodified by the protein demodifying activity to be determined, and wherein modification or demodification of said site of modification affects cleavage of the said site of activation or inactivation by the said protease.

20. A detection enzyme construct according to claim 19, comprising a site of modification and a site of activation, wherein activation of the detection enzyme construct results from cleavage of said site of activation by an activation protease to form active detection enzyme, wherein said site of modification of the detection enzyme construct is capable of being modified by the protein modifying activity to be determined or demodified by the protein demodifying activity to be determined, and wherein modification or demodification of said site of modification determines whether said activation protease can cleave the said site of activation.

21. A detection enzyme construct according to claim 19, comprising a site of modification, a site of activation and a site of inactivation, wherein activation of the detection enzyme construct results from cleavage of said site of activation by an activation protease to form active detection enzyme, and inactivation of the detection enzyme construct or detection enzyme results from cleavage of said site of inactivation by a deactivation protease, wherein said site of modification of the detection enzyme construct is capable of being modified by the protein modifying activity to be determined or demodified by the protein demodifying activity to be determined, and wherein modification or demodification of said site of modification determines whether said activation protease can cleave the said site of activation or said inactivation protease can cleave the said site of inactivation.

22. A detection enzyme construct according to claim 21, wherein modification or demodification of said site of modification determines whether said inactivation protease can cleave the said site of inactivation.

23. A detection enzyme construct according to any one of claims 19-22, wherein active detection enzyme is detectable by its conversion of a substrate of the detection enzyme to a reaction product.

24. A detection enzyme construct according to any one of claims 19-23, derived from a protease enzyme or protease pro-enzyme.

25. A detection enzyme construct according to claim 24, derived from a serine protease enzyme or serine protease pro-enzyme or from a cysteine protease enzyme or cysteine protease pro-enzyme.

26. A detection enzyme construct according to any one of claims 19-23, derived from a pro-enzyme selected from the group consisting of pro-urokinase, pro-granzyme, pro-caspase-3 and pro-caspase-7.

27. A detection enzyme construct according to any one of claims 19-23, derived from pro-urokinase.

28. A detection enzyme construct according to any one of claims 19-23, derived from pro-caspase-3.

29. A detection enzyme construct according to any one of claims 19-28, which contains an artificial modification site, or an artificial activation cleavage site, or an artificial inactivation cleavage site, or any combination thereof, and optionally further changes, introduced into a protease enzyme or protease pro-enzyme in such a way that modification or demodification of said modification site affects activatability of the detection enzyme construct by treatment with said activation protease or inactivatability of the detection enzyme construct or detection enzyme by treatment with said inactivation protease.

30. A detection enzyme construct according to claim 29, wherein the activation protease is selected from the group consisting of (a) the natural activation protease of said protease pro-enzyme, (b) a protease specifically cleaving an artificial activation cleavage site introduced into said protease pro-enzyme, (c) an aminopeptidase, and (d) a carboxypeptidase.

31. A detection enzyme construct according to claim 29, wherein the inactivation protease is the same or different from the activation protease and is selected from the group consisting of (a) a natural inactivation protease of said protease enzyme, (b) a protease specifically cleaving an artificial inactivation cleavage site introduced into said protease pro-enzyme, (c) an aminopeptidase, and (d) a carboxypeptidase.

32. A cell expressing or containing a detection enzyme construct as defined in any one of claims 19-31.

33. A kit for determining a protein modifying activity or a protein demodifying activity in a sample, comprising a detection enzyme construct as defined in any one of claims 19-31, or a cell as defined in claim 32, and further comprising at least one member of the group consisting of activation protease, inactivation protease, substrate of the detection enzyme, a standard composition, a buffer composition, an antibody composition, a stabilizing composition, an assay plate or container, and instructions for use.

34. An expression vector coding for a detection enzyme construct as defined in any one of claims 19-31.
